# DEMANDE DE BREVET EUROPEEN

(11) **EP 2 345 744 A2**
(43) Date de publication de la demande: **20.07.2011**
(21) Numéro de dépôt: 10184553.5
(22) Date de dépôt: 18.01.2006
(51) Int. Cl.: C12Q 1/68

(54) **Methode de detection, non invasive, prenatale, in vitro de l'etat sain normal, de l'etat de porteur sain ou de l'etat de porteur malade de la mucoviscidose**

(30) Priorité: 18.01.2005 FR 0500512
(62) Demande divisionnaire de: 06709125.6
(71) Demandeur: INSTITUT NATIONAL DE LA SANTE ET DE LA RECHERCHE MEDICALE, 75654 Paris Cedex 13 (FR); ASSISTANCE PUBLIQUE, HOPITAUX DE PARIS, 75004 Paris (FR); UNIVERSITE RENE DESCARTES - PARIS V, 75006 Paris (FR)
(72) Inventeur: Paterlini, Patrizia, 75015, Paris (FR); Bonnefont, Jean-Paul, 92700, Colombes (FR); Desitter, Isabelle, 75016, Paris (FR); Saker, Ali, 94550, Chevilly Larue (FR)
(74) Mandataire: Desaix, Anne

(57) **Abrégé**

La présente invention est relative à une méthode de détection, non invasive, prénatale *in vitro* de l'état sain normal, de l'état de porteur sain ou de l'état de porteur malade de la mucoviscidose, à partir de cellule(s) foetale(s) issue(s) d'un échantillon maternel, comprenant l'ADN à tester d'un individu. L'invention porte également sur des amorces oligonucléotidiques et sur leur utilisation dans le cadre d'une méthode de détection, non invasive, prénatale *in vitro* de l'état de porteur sain ou de porteur malade de la mucoviscidose.

## Description

La présente invention est relative à une méthode de détection, non invasive, prénatale de l'état sain normal, de l'état de porteur sain ou de l'état de porteur malade de la mucoviscidose, à partir de cellule(s) foetale(s) issues d'un échantillon maternel, comprenant l'ADN à tester d'un individu. La méthode selon l'invention est utilisable dans un protocole de diagnostic *in vitro* de la mucoviscidose.

L'invention porte également sur des amorces oligonucléotidiques et sur leur utilisation dans le cadre d'une méthode de détection, non invasive, prénatale de l'état sain normal, de l'état de porteur sain ou de l'état de porteur malade de la mucoviscidose. L'invention concerne aussi des amorces et leur utilisation dans le cadre d'une méthode pour l'identification, sur une préparation d'ADN dérivée du génome d'au moins une cellule collectée à partir d'un échantillon maternel, d'un ou plusieurs marqueurs de polymorphisme génétique, démontrant la contribution bi-parentale à l'ADN et, en conséquence, l'origine foetale de ladite au moins une cellule.

L'invention porte également sur un polynucléotide et une association de polynucléotides utilisables comme amorces pour amplifier une quantité d'ADN d'un échantillon biologique, ainsi que sur un kit pour la détection, non invasive, prénatale de la mucoviscidose.

La mucoviscidose est la maladie récessive autosomique mettant en danger la vie des malades la plus répandue parmi les enfants blancs, affectant un nouveau-né sur 3500 aux Etats-Unis (Kosorok, Disease, 1996), avec une fréquence des porteurs variant de 1 sur 20 à 1 sur 40 ou plus (Bobadilla, 2002). La maladie est caractérisée par une imperfection du canal chlore qui est imputable à des altérations, précisément à des mutations dans le gène régulateur de la conductance transmembranaire de la mucoviscidose (CFTR) (Cystic Fibrosis Foundation, 2003). Environ 1000 mutations ont été découvertes, notamment celle correspondante à l'allèle deltaF508 qui représente 68% des allèles mutés dans le monde (Karem, 1989).

L'imperfection du canal chlore entraîne des anomalies au niveau des électrolytes et des sécrétions macromoléculaires des glandes exocrines, induisant un risque d'obstruction canalaire pancréatique in utero, d'insuffisance pancréatique, de bronchopneumopathie chronique obstructive et d'infections respiratoires récurrentes. L'espérance de vie moyenne des patients est d'environ 30 ans, mais la survie des patients atteints de mucoviscidose a augmenté lors des 4 dernières décennies.

Plus de 10 millions d'américains portent la mutation delta F508 du gène CFTR associée à la mucoviscidose et environ 80% des nourrissons nés avec la mucoviscidose sont conçus par des parents qui n'ont pas d'antécédents familiaux de la maladie (Fink, Collins, 1997). Comme la maladie est relativement commune, ses premiers signes sont précoces. Les coûts de traitement de cette maladie sont élevés, elle est finalement fatale. La mucoviscidose fait partie des maladies les plus prometteuses pour un criblage génétique (Balinsky, 2004, Garber, Fenerty, 1991).

Un diagnostic génétique fiable est réalisé sur des cellules foetales obtenues par amniocentèse, prélèvement de villosités choriales (PVC) ou prise de sang foetal, lesquels comportent un risque non négligeable de fausse couche respectivement égal à 0,5%, 1% et 3% (Ciarleglio, 2003). Il y a donc un intérêt à rechercher des méthodes de diagnostic alternatives, de préférence non invasives.

La demande de brevet WO02/088736 décrit une méthode de diagnostic prénatal non-invasive mise en oeuvre à partir d'un échantillon de sang maternel permettant en particulier la détection précoce du sexe génétique du foetus. L'enseignement de cette demande de brevet est incorporé par référence dans la présente demande.

Des efforts antérieurs pour enrichir les cellules foetales circulantes et les utiliser à des fins de diagnostic prénatal de la mucoviscidose ont été réalisés (Martel-Petit, 2001), et l'ADN foetal issu du plasma maternel a été utilisé pour détecter une mutation paternelle héréditaire (Gonzalez-Gonzalez, 2002).

Aucune méthode pour le diagnostic de la mucoviscidose ne s'est cependant révélée fiable et suffisamment efficace pour être appliquée de façon routinière en remplacement des protocoles invasifs.

Les inventeurs se sont intéressés à une méthode de détection, non invasive, prénatale de la mucoviscidose, fiable et suffisamment efficace pour être appliquée de façon routinière, laquelle n'implique pas de prélèvement de tissus ou cellules foetales par biopsie et/ou effraction de la barrière placentaire.

L'invention définit des moyens adaptés à la détection d'anomalies génétiques connues, en particulier de mutations connues du gène CFTR, ou des moyens adaptés à la détection de mutations inconnues ou indéterminées du gène CFTR.

Dans le cadre de l'invention, les cellules foetales sont prélevées à partir d'un échantillon prélevé chez la femme enceinte.

L'invention a donc pour objet une méthode de détection, non invasive prénatale, *in vitro* de l'état sain normal, de l'état de porteur sain ou de l'état de porteur malade de la mucoviscidose, à partir d'un échantillon de cellule(s) foetate(s) isolée(s) d'un échantillon maternel prélevé comprenant l'ADN à tester d'un individu, ladite méthode comprenant les étapes suivantes :
a. l'enrichissement en cellules foetales d'un échantillon maternel prélevé, pur ou dilué, susceptible de comprendre des cellules d'origine foetale;
b. l'analyse des cellules retenues et la sélection des cellules présumées d'origine foetale ;
c. la démonstration par analyse génétique, de l'origine foetale d'une ou plusieurs cellules sélectionnées à l'étape b et,
d. sur l'ADN foetal de cellule(s) sélectionnée(s) à l'étape c., la recherche d'allèles du gène CFTR porteurs de la mutation ΔF508 ou d'autres mutations connues ou, la recherche d'allèles d'un locus portant un polymorphisme génotypique génétiquement lié à une mutation morbide non identifiée du gène CFTR, au moyen des étapes suivantes:
   - l'amplification de l'ADN foetal au moyen de couples d'amorces choisis pour leur capacité à amplifier le locus susceptible de porter la mutation connue recherchée sur le gène CFTR ou un locus comprenant un polymorphisme génotypique génétiquement lié (linkage) à la mutation du gène CFTR lors de la ségrégation,
   - l'identification sur les allèles correspondant aux fragments d'ADN amplifiés, de la présence ou de l'absence de la mutation connue recherchée du gène CFTR ou du locus polymorphique génétiquement lié au gène CFTR et
   - la comparaison des allèles foetaux avec les allèles correspondant d'échantillons contrôle et la détermination à partir de l'observation des allèles amplifiés, de la détection de l'état sain normal, de l'état de porteur sain ou de l'état de porteur malade de la mucoviscidose chez l'individu testé.

Dans le cadre de la présente demande, l'individu est l'individu à naître représenté pour les besoins de la détection selon l'invention, par ses cellules foetales. La méthode de détection selon l'invention offre des performances suffisantes en termes de spécificité et de sensibilité pour être considérée comme une méthode de diagnostic prénatal alternative aux méthodes invasives, ou à tout le moins pour être inclue dans des protocoles de diagnostic.

La méthode selon l'invention permet ainsi, à partir de l'ADN d'une ou d'un petit nombre de cellules foetales, d'identifier la présence ou l'absence, hétérozygote ou homozygote, d'allèles portant des anomalies du gène CFTR responsables, chez l'individu né, de la mucoviscidose.

La présence d'allèles homozygotes mutés au niveau du gène CFTR rend compte d'un individu qui sera normal, porteur sain ou affecté (porteur malade) par la mucoviscidose.

Il existe quatre statuts:
- homozygote sain : 2 allèles normaux ;
- hétérozygote simple : 1 allèle normal / 1 allèle muté (deltaF508 ou mutation inconnue) ;
- homozygote atteint : 2 allèles mutés (deltaF508)
- hétérozygote composite :
   soit 1 allèle muté deltaF508 / 1 allèle muté mutation inconnue
   soit 1 allèle muté mutation inconnue / 1 allèle muté mutation inconnue.

Il y a absence de la maladie dans les cas « homozygote sain » et « hétérozygote simple ».

Il y a présence de la maladie dans les cas « homozygote atteint » et « hétérozygote composite ».

L'expression « état de porteur sain » signifie que l'individu est hétérozygote simple pour l'allèle muté du gène CFTR.

L'expression « état de porteur malade » signifie que l'individu est homozygote atteint ou hétérozygote composite pour l'allèle muté du gène CFTR.

L'expression « état sain normal » signifie que l'individu comporte deux allèles normaux.

Cette détection est réalisée par une méthode de recherche directe d'une mutation connue, ou lorsque la mutation du gène CFTR responsable de la mucoviscidose n'est pas connue ou n'a pas été identifiée chez les parents de l'individu testé, par une méthode indirecte, consistant à détecter un ou plusieurs marqueurs de polymorphisme ayant un lien génétique (linkage) avec le gène CFTR, ledit marqueur se trouvant être hérité par co-ségrégation avec ledit gène CFTR, au cours de générations.

Dans le cadre de cette méthode de détection, non invasive, prénatale *in vitro* de la mucoviscidose, une altération en particulier une mutation connue du gène CFTR, est par exemple celle qui affecte la position 508 des acides aminés correspondants, résultant en la délétion de trois nucléotides identifiée sous la désignation delta F508.

Lorsque la méthode selon l'invention est mise en oeuvre pour rechercher une mutation inconnue ou qui n'a pas été identifiée chez les parents de l'individu testé, on a recours à la recherche de polymorphismes génétiques, en particulier de polymorphismes génotypiques situés en amont ou en aval du gène CFTR, sur le chromosome 7, qui sont caractérisés par leur coségrégation avec le gène CFTR au cours des générations. Un allèle d'un tel marqueur est en principe toujours associé au même allèle du gène CFTR.

Par l'expression « échantillon de contrôle » on entend, par rapport à l'échantillon biologique donnant accès aux cellules foetales, un ou plusieurs échantillons biologiques provenant respectivement du père et de la mère lorsque la mutation recherchée du gène CFTR est une mutation connue, par exemple la mutation du locus delta F508. Lorsque la mutation n'est pas connue ces échantillons de contrôle sont un ou plusieurs échantillons biologiques provenant respectivement du père et de la mère et on doit y ajouter un échantillon biologique d'un enfant de la même fratrie qui est affecté par la mucoviscidose (cas index). Ces échantillons sont traités pour donner accès à l'ADN génomique respectivement du père, de la mère et de l'enfant de la même fratrie.

Par l'expression « échantillon maternel», on entend tout échantillon biologique, prélevé chez la mère de l'individu à tester, par une méthode non invasive, ledit échantillon comprenant des cellules foetales ainsi que d'autres types de cellules.

Dans un exemple spécifique, l'échantillon maternel peut être du sang maternel prélevé chez la femme enceinte, comprenant des cellules foetales ainsi que d'autres cellules circulantes, telles que les leucocytes. L'étape a de la méthode selon l'invention, consistera dans ce cas à séparer les cellules foetales des autres cellules circulantes.

Dans un autre mode de réalisation de l'invention, l'échantillon maternel est prélevé chez la femme enceinte au niveau du col de l'utérus, soit par lavage avec une solution (par exemple physiologique), soit par prélèvement de glaire sans lavage, sans effraction du sac amniotique. L'échantillon ainsi prélevé contient en principe des cellules foetales.

La méthode de détection de la mucoviscidose selon l'invention peut être entendue comme une méthode *in vitro* non invasive permettant d'évaluer la prédisposition d'un individu à la mucoviscidose, en identifiant chez cet individu la présence ou l'absence, hétérozygote ou homozygote, d'allèles portant des anomalies du gène CFTR responsables de la mucoviscidose.

Sauf à disposer des informations requises sur l'ADN des échantillons de contrôle avant d'entreprendre la détection selon l'invention sur l'ADN foetal prélevé, les échantillons de contrôle sont traités, pour les étapes c. et d. de la méthode de l'invention, comme l'échantillon d'ADN foetal.

Dans un mode de réalisation particulier de l'invention, la quantité d'ADN qui est amplifiée est une petite quantité d'ADN, avantageusement inférieure à 5pg, par exemple de l'ordre de 2pg. Les amorces d'amplification doivent donc être définies pour permettre l'amplification d'une petite quantité d'ADN, par exemple une quantité d'ADN correspondant à celle d'une cellule unique ou un nombre réduit de cellules foetales.

Selon un exemple spécifique de la méthode de détection, l'étape d'amplification de l'étape c. et/ou l'étape d'amplification de l'étape d. comprend au moins une phase d'amplification, par exemple, elle comprend une première phase d'amplification réalisée avec des amorces externes et une deuxième phase d'amplification réalisée avec des amorces internes.

Dans un exemple spécifique de l'invention, l'ADN de l'échantillon de l'individu testé dérive du génome d'au moins une cellule foetale.

Dans un exemple spécifique, l'ADN de l'échantillon cellulaire dérive du génome d'au moins une cellule foetale isolée, en particulier du génome d'un petit nombre de cellules, allant de 1 à 20 cellules, plus particulièrement de 1 à 10 cellules, par exemple le(s) génome(s) d'1, 2 ou 3 cellules, préférentiellement au moins trois cellules (cf. exemple 4 ci-dessous) et avantageusement du génome de cellules isolées individuellement.

Dans un exemple spécifique, l'ADN de l'échantillon dérive du génome d'une cellule foetale unique ou d'un nombre de cellules foetales égal ou inférieur à 20, plus particulièrement à 10. De préférence, le nombre de cellules foetales est au moins de trois cellules.

Si la détection sur l'ADN d'une cellule unique n'offre pas un résultat pertinent, en particulier si l'amplification est réalisée par PCR et que l'un des deux allèles du gène portant le marqueur recherché n'est pas amplifié ou pas détecté (situation dite d'Allele Drop Out) du fait de la quantité d'ADN mise en oeuvre, on peut augmenter le nombre de cellules foetales à partir desquelles on récupère l'ADN à tester qui est alors utilisé sous forme de pool. En particulier, on aura recours à 2, 3, ou par exemple entre 2 et 20 cellules, préférentiellement au moins trois cellules, avantageusement entre 3 et 20 cellules (bornes incluses), cf. exemple 4 ci-dessous (absence d'ADO).

Après avoir déterminé les allèles du gène CFTR de l'ADN foetal ou les allèles du locus portant le ou les marqueurs de polymophisme ayant un linkage avec le gène CFTR, une comparaison est réalisée avec les allèles du père, de la mère et lorsque cela s'avère nécessaire, de l'enfant de la fratrie affecté par la mucoviscidose (appelé Cas Index) afin de savoir si, dans l'échantillon d'ADN étudié de l'individu testé, il y a présence homozygote d'un allèle caractéristique d'une altération génétique liée à la mucoviscidose, présence hétérozygote d'un allèle caractéristique d'une altération génétique liée à la mucoviscidose ou absence homozygote d'un allèle caractéristique d'une altération génétique liée à la mucoviscidose.

Pour pouvoir détecter *in vitro* la mucoviscidose chez l'individu dont on teste l'ADN foetal, il faut, dans une première phase de la détection, conformément à l'étape c. de la méthode de l'invention, établir la démonstration de l'origine foetale de l'ADN. Cette détermination du caractère foetal de l'ADN est encore appelée génotypage dans la présente demande. Cette étape est réalisée au moyen de marqueurs de polymorphisme génétique. Il est nécessaire que les marqueurs de polymorphisme génétique utilisés soient informatifs, en ce sens qu'ils permettent de différencier l'allèle de chaque marqueur apporté par le père et celui apporté par la mère. Il faut donc que les marqueurs de polymorphisme maternel et paternel soient distincts l'un de l'autre.

Dans un mode de réalisation particulier, l'invention concerne une méthode de détection de l'état de porteur sain ou de porteur malade de la mucoviscidose, caractérisée en ce que les amorces informatives utilisées à l'étape c. sont dérivées de la séquence d'un chromosome choisi parmi le chromosome 16, le chromosome 21 ou le chromosome 7.

Selon un mode de réalisation particulier de l'invention, les amorces utilisées à l'étape c. sont dérivées de la séquence du chromosome 7 et sont proches de l'allèle morbide de la mucoviscidose. L'allèle morbide désigné ici est l'allèle du gène CFTR (situé sur le chromosome 7) qui porte l'anomalie responsable de la mucoviscidose.

Préalablement à l'étape de génotypage c. et à l'étape de recherche des allèles du gène CFTR ou des allèles d'un locus portant un polymorphisme génétique génétiquement lié à un allèle anormal du gène CFTR, l'invention requiert la mise en oeuvre des étapes suivantes :
a. l'enrichissement en cellules foetales d'un échantillon maternel prélevé, pur ou dilué, susceptible de comprendre des cellules d'origine foetale;
b. l'analyse des cellules retenues pendant l'étape a) afin d'obtenir une présomption de leur origine foetale ou maternelle ;

Dans le cadre de la méthode de détection, non invasive, prénatale de la mucoviscidose selon l'invention, tout type de technique peut être utilisé, d'une part, pour l'enrichissement en cellules foetales d'un échantillon maternel prélevé, pur ou dilué, susceptible de comprendre des cellules d'origine foetale;(étape a)), d'autre part, pour l'analyse des cellules retenues pendant l'étape a) afin d'obtenir une présomption de leur origine foetale ou maternelle (étape b)).

Différentes méthodes utilisables pour ces étapes a. et b. seront envisagées et illustrées ci-dessous, après la description des étapes c. et d. Dans un exemple spécifique de l'invention, les génomes cellulaires sont analysés individuellement au cours de l'étape c.. Au cours de l'étape d., l'analyse des génomes cellulaires est soit effectuée sur le génome de chaque cellule pris individuellement, soit sur les génomes réunis (poolés) de plusieurs cellules foetales.

Plus particulièrement, l'étape c. de génotypage comprend ou consiste en l'identification, sur une préparation d'ADN dérivée du génome de la cellule unique collectée (cellule individuelle), d'un ou plusieurs marqueurs de polymorphisme génétique, ou d'une combinaison de ces marqueurs, démontrant la contribution bi-parentale à l'ADN et en conséquence, l'origine foetale de ladite au moins une cellule.

Dans le cadre de la présente méthode de détection, non invasive, prénatale de la mucoviscidose, par marqueur de polymorphisme génétique, il faut comprendre toute caractéristique identifiable sur l'ADN, dont la présence est corrélée à un génotype particulier et le cas échéant, à un phénotype particulier. Les marqueurs utilisés au cours de l'étape c. permettent de distinguer l'ADN paternel de l'ADN maternel et donc de démontrer la composition bi-parentale de l'ADN foetal. Il peut donc être suffisant qu'ils soient corrélés à un génotype particulier.

On peut citer par exemple les marqueurs de polymorphisme de longueur de fragments de restriction (RFLP), les marqueurs SNP (Single Nucleotide Polymorphism), les marqueurs micro-satellites, les marqueurs VNTR (Variable Number of Tandem Repeats) ou STR (Short Tandem Repeats).

Les marqueurs micro-satellites sont particulièrement préférés pour la caractérisation des cellules et la mise en oeuvre du diagnostic prénatal. Dans un mode de réalisation de l'invention, au moins un marqueur de polymorphisme à identifier est un marqueur microsatellite, un marqueur VNTR (Variable Number of Tandem Repeats), un marqueur SNP (Single Nucleotide Polymorphism) ou un marqueur STR (Short Tandem Repeats). Ceux-ci présentent en effet l'avantage d'être identifiables par l'amplification à l'aide d'amorces spécifiques. Les marqueurs micro-satellites, VNTR ou STR, sont composés de répétitions en tandem, le plus souvent de motifs polyCA/GT. Les variations alléliques, dues à la variation du nombre des répétitions, sont aisément détectées par amplification de type PCR, grâce à l'utilisation d'amorces correspondant aux séquences uniques flanquant le microsatellite. Une carte physique de ces marqueurs microsatellites et la séquence de leurs amorces associées sont décrits par Dib *et al.* (Dib, C., Faure, S., Fizames, C., Samson, D., Drouot, N., Vignal, A., Missasseau, P., Marc, S., Hazan, J., Seboun, E., Lathrop, M., Gyapay, G., Morissette, J., and Weissenbach, J. A comprehensive genetic map of the human genome based on 5.264 microsatelites. Nature 1996 380 : 152-154). Par cette méthodologie, la mise en évidence d'une contribution biparentale au génotype des cellules analysées permet d'établir de manière certaine l'origine foetale des cellules analysées.

Afin de démontrer l'origine foetale ou au contraire maternelle d'une cellule unique collectée, dans un mode de mise en oeuvre particulier, il peut être suffisant d'effectuer la recherche d'un marqueur ou d'une combinaison de marqueurs ou un dosage allélique de ces marqueurs qui se distinguent de ceux du génome de cellules maternelles. En particulier la recherche sur le génome de ladite cellule collectée, peut être portée sur un marqueur ou d'une combinaison de marqueurs spécifique de l'ADN de cellules paternelles. Leur présence est nécessairement la signature d'une origine foetale de la cellule en cause.

Selon un mode de réalisation particulier de l'invention, l'étape c. peut comprendre :
i) l'amplification de l'ADN pris individuellement, d'une ou plusieurs cellules sélectionnées, à l'aide d'amorces dites informatives, capables d'amplifier des polymorphismes génétiques préalablement déterminés pour distinguer les allèles maternels des allèles paternels de façon à reconnaître le génome foetal par la présence d'un allèle paternel et d'un allèle maternel dans chaque cellule sélectionnée ;
ii) la comparaison des allèles de l'ADN desdites cellules avec les allèles parentaux correspondants;
iii) la sélection de l'ADN de cellule(s) comportant un allèle maternel et un allèle paternel pour le ou les polymorphismes génétiques identifiés, démontrant l'origine foetale de l'ADN de la(les) cellule(s).

Les conditions de réalisation des amplifications sont par exemple les suivantes : 5 min 94°C, 40x (30 s, 94°C; 30 à 45 s, 55 à 61 °C ; 30 s 72°C), 5 min 72°C.

Dans un exemple spécifique, deux phases d'amplification peuvent être réalisées comme suit : une première phase d'amplification de la préparation d'ADN dérivée du génome d'une cellule unique collectée est effectuée avec une ou plusieurs amorces nucléotidiques dites externes par rapport à la séquence cible et une deuxième phase d'amplification sur le produit d'amplification obtenu par l'étape précédente est effectuée avec une ou plusieurs amorces nucléotidiques dites internes ou emboîtées par rapport aux dites amorces externes. Les amorces externes et internes ci-dessus, sont dites informatives, c'est à dire capables d'amplifier des polymorphismes génétiques préalablement déterminés comme étant distincts et caractéristiques sur des allèles maternels d'une part et paternel d'autre part, de façon à permettre d'identifier les polymorphismes génétiques recherchés typiques des allèles foetaux.

Les amorces sont des oligonucléotides synthétiques dont la séquence est déterminée pour permettre l'amplification d'une séquence d'ADN comprenant les marqueurs informatifs du caractère foetal de la cellule testée.

En conséquence, les amorces utilisables pour réaliser l'étape c. sont dites informatives du caractère foetal de la cellule ou, selon un mode de réalisation particulier de l'invention, ces amorces sont dites informatives de la contribution bi-parentale à la cellule testée.

Des amorces informatives conduisent avantageusement à amplifier des fragments d'ADN comportant plusieurs polymorphismes, de façon préférée un grand nombre de polymorphismes. Les polymorphismes sont identifiés sur les séquences d'ADN d'intérêt, notamment sur le chromosome 7, dans les bases de données disponibles (NCBI par exemple). A partir de ces données, on identifie des oligonucléotides qui permettent l'amplification dans les conditions de l'invention, de façon à permettre le génotypage ou la détection efficace, sensible et spécifique.

Ces amorces informatives peuvent être identifiées spécifiquement pour une famille (père et mère de l'individu testé) par une analyse génétique comparée des génomes du père et de la mère.

Alternativement, ces amorces informatives peuvent être des amorces dont on a montré qu'elles sont capables d'amplifier des séquences d'ADN comprenant des marqueurs de polymorphisme génétique partagés par plusieurs familles, voir consensuels, pour ce qui est du caractère informatif de l'origine paternelle ou maternelle d'un allèle d'un gène.

Tel est le cas des amorces identifiées ci-dessous qui ont permis de révéler la contribution bi-parentale à l'ADN de la cellule testée pour démontrer son origine foetale.

Les amorces externes dites amorces informatives utilisables à l'étape c. sont choisies parmi les couples d'amorces suivants où F signifie « forward » et s'hybride au brin d'ADN orienté 3' - 5' dans la séquence cible et R signifie « reverse », et s'hybride au brin d'ADN orienté 5' - 3' dans la séquence cible:
- F: 5'-AAAAACCCTGGCTTATGC-3' (SEQ ID NO : 1) ; R: 5'-AGCTACCATAGGGCTGGAGG-3' (SEQ ID NO : 2) ;
- F: 5'-GGAATCTGTTCTGGCAATGGAT-3' (SEQ ID NO : 5); R: 5'-TTGCAATGAGCCGAGATCCTG-3' (SEQ ID NO : 6) ;
- F: 5'-CAGATGCTCGTTGTGCACAA-3' (SEQ ID NO : 9); R: 5'-ATACCATTTACGTTTGTGTGTG-3' (SEQ ID NO : 10) ;
- F : 5'-TGACAGTGCAGCTCATGGTC-3' (SEQ ID NO : 13); R: 5'-GGTCATTGGTCAAGGGCTGCT-3' (SEQ ID NO : 14) ;
- F: 5'-TTGACATTCTTCTGTAAGGAAGA-3' (SEQ ID NO : 17); R: 5'-AGGCTTGCCAAAGATATTAAAAG-3' (SEQ ID NO : 18);
- F: 5'-TTGTGAATAGTGCTGCAATG-3' (SEQ ID NO: 21) ; R: 5'-ATGTACACTGACTTGTTTGAG-3' (SEQ ID NO : 22) ;
- F: 5'- AAGTAATTCTCCTGCCTCAG-3' (SEQ ID NO: 29); R: 5'-AGCTACTTGCAGTGTAACAGCATTT -3' (SEQ ID NO: 30) ;
- F: 5'- GAATTATAACCGTAACTGATTC-3' (SEQ ID NO: 33) ; R:5'-GAGATAATGCTTGTCTGACTTC-3' (SEQ ID NO: 34).

Les amorces internes ou emboîtées dites amorces informatives utilisées à l'étape c. sont choisies parmi les couples d'amorces suivants :
- F: 5'-CTTGGGGACTGAACCATCTT-3' (SEQ ID NO: 3); R: 5'-AGCTACCATAGGGCTGGAGG-3' (SEQ ID NO : 4) ;
- F : 5'-AAAGGCCAATGGTATATCCC-3' (SEQ ID NO : 7); R: 5'-GCCCAGGTGATTGATAGTGC-3' (SEQ ID NO : 8) ;
- F: 5'-GATCCCAAGCTCTTCCTCTT-3' (SEQ ID NO : 11); R: 5'-ACGTTTGTGTGTGCATCTGT-3' (SEQ ID NO : 12) ;
- F: 5'-GGATAAACATAGAGCGACAGTTC-3' (SEQ ID NO : 15); R: 5'-AGACAGAGTCCCAGGCATT-3' (SEQ ID NO : 16) ;
- F: 5'-CCCTCTCAATTGTTTGTCTACC-3' (SEQ ID NO : 19); R: 5'-GCAAGAGATTTCAGTGCCAT-3' (SEQ ID NO : 20) ;
- F: 5'-ATGTACATGTGTCTGGGAAGG-3' (SEQ ID NO : 23); R: 5'-TTCTCTACATATTTACTGCCAACA-3' (SEQ ID NO : 24) ;
- F: 5'- CCTTGGGCCAATAAGGTAAG-3' (SEQ ID NO: 31); R: 5'-AGCTACTTGCAGTGTAACAGCATTT-3' (SEQ ID NO:32) ;
- F: 5'- CTGATTCATAGCAGCACTTG -3' (SEQ ID NO: 35) ; R:5'-AAAACATTTCCATTACCACTG-3' (SEQ ID NO:36).

L'étape d. qui suit la réalisation de l'étape c. doit permettre de détecter la mucoviscidose à partir des cellules foetales retenues à l'issue de l'étape c.. On recherche donc des mutations connues du gène CFTR ou des marqueurs de polymorphisme formant un linkage avec l'allèle muté du gène CFTR, dont la présence témoigne d'altérations de l'ADN du gène CFTR.

On sait dans l'art antérieur, que ces mutations affectent le gène CFTR, majoritairement dans la région dite du locus F508 où une mutation par délétion de trois nucléotides conduit à l'obtention d'un gène CFTR muté en position « 508 ». En conséquence, des fragments amplifiés du gène CFTR, au moyen des amorces selon l'invention, sont ceux qui englobent la mutation ΔF508. Les inventeurs ont donc défini des oligonucléotides, utilisables en tant qu'amorces d'amplification pour effectuer l'étape d., qui permettent d'amplifier une séquence d'ADN comportant le locus F508, et de détecter la mutation dudit locus.

D'autres mutations peuvent cependant avoir lieu sur le gène CFTR, dans d'autres régions que le locus F508. Les inventeurs ont donc défini d'autres oligonucléotides amorces, capables d'amplifier des séquences d'ADN portant un polymorphisme génétique formant un linkage (ou liaison génétique) avec un locus portant d'autres mutations dites « non identifiées » associées à la mucoviscidose. Cette amplification se fait dans le cadre de la méthode de détection de la mucoviscidose. Ces amorces ont été identifiées à partir de l'ADN du chromosome 7.

L'étape d. de la méthode de l'invention peut donc être mise en oeuvre en utilisant des couples d'amorces choisis parmi les suivants:
- pour les mutations de type ΔF508:
   - le couple d'amorces externes DeltaF508 out (F: 5'-TGGAGCCTTCAGAGGGTAAA-3'(SEQ ID NO: 25) ; R: 5'-TGCATAATCAAAAAGTTTTCACA-3' (SEQ ID NO : 26)), utilisé seul ou lors de la première phase d'amplification de l'étape d.quand elle est effectuée, et
   - le couple d'amorces internes deltaF508 in (F : 5'-TCTGTTCTCAGTTTTCCTGG-3' (SEQ ID NO : 27); R: 5'-TCTTACCTCTTCTAGTTGGC-3' (SEQ ID NO : 28)) utilisé lors de la deuxième phase d'amplification de l'étape d. ou seul en cas d'amplification unique à l'étape d. ;
- pour les autres mutations indéterminées du locus deltaF508, des amorces identifiées sur la chromosome 7 amplifiant un fragment d'ADN formant un linkage avec le gène CFTR:
   - F : 5'-AAAAACCCTGGCTTATGC-3' (SEQ ID NO : 1); R : 5'-AGCTACCATAGGGCTGGAGG-3' (SEQ ID NO : 2) ;
   - F : 5'-GGAATCTGTTCTGGCAATGGAT-3' (SEQ ID NO : 5) ; R :
      5'-TTGCAATGAGCCGAGATCCTG-3' (SEQ ID NO : 6) ;
   - F: 5'- AAGTAATTCTCCTGCCTCAG-3' (SEQ ID NO: 29) ; R : 5'-AGCTACTTGCAGTGTAACAGCATTT -3' (SEQ ID NO: 30) ;
   - F: 5'- GAATTATAACCGTAACTGATTC-3' (SEQ ID NO: 33) ; R:5'-GAGATAATGCTTGTCTGACTTC-3' (SEQ ID NO: 34) ;
   - F : 5'-CTTGGGGACTGAACCATCTT-3' (SEQ ID NO : 3) ; R : 5'-AGCTACCATAGGGCTGGAGG-3' (SEQ ID NO : 4) ;
   - F : 5'-AAAGGCCAATGGTATATCCC-3' (SEQ ID NO : 7); R : 5'-GCCCAGGTGATTGATAGTGC-3' (SEQ ID NO : 8) ;
   - F: 5'- CCTTGGGCCAATAAGGTAAG-3' (SEQ ID NO: 31); R : 5'-AGCTACTTGCAGTGTAACAGCATTT-3' (SEQ ID NO: 32) ;
   - F: 5'- CTGATTCATAGCAGCACTTG -3' (SEQ ID NO: 35) ; R:5'-AAAACATTTCCATTACCACTG-3' (SEQ ID NO: 36).

Selon un mode de réalisation particulier de l'invention, dans le cadre de la méthode de détection, non invasive, prénatale de la mucoviscidose selon la voie dite indirecte, dans laquelle l'anomalie du gène CFTR recherchée n'est pas connue, les étapes c et d sont réalisées dans les conditions suivantes :
- l'amplification selon les étapes c. et d. est confondue et réalisée au moyen d'amorces informatives d'amplification capables d'amplifier un locus comprenant un polymorphisme génotypique génétiquement lié (linkage) au gène CFTR lors de la ségrégation, par exemple les amorces identifiées dans la présente demande sur le chromosome 7 et,
- la comparaison avec les échantillons contrôles à l'étape d. comporte la comparaison des allèles identifiés de l'ADN foetal avec les allèles correspondants paternels, les allèles correspondants maternels et les allèles correspondants d'un enfant de la fratrie affecté par la mucoviscidose.

Selon ce mode de détection dit indirect, dans le cadre de l'invention, l'au moins une phase d'amplification de l'ADN foetal, de l'ADN maternel, de l'ADN paternel et de l'ADN de l'enfant de la fratrie est réalisée avec un ou plusieurs couples d'amorces choisis parmi :
(F : 5'-AAAAACCCTGGCTTATGC-3' SEQ ID NO : 1 ; R: 5'-AGCTACCATAGGGCTGGAGG-3' SEQ ID NO : 2),
(F : 5'-GGAATCTGTTCTGGCAATGGAT-3' SEQ ID NO : 5 ; R: 5'-TTGCAATGAGCCGAGATCCTG-3' SEQ ID NO : 6),
(F: 5'- AAGTAATTCTCCTGCCTCAG-3' (SEQ ID NO: 29); R: 5'-AGCTACTTGCAGTGTAACAGCATTT -3' (SEQ ID NO: 30)) ;
(F: 5'- GAATTATAACCGTAACTGATTC-3' (SEQ ID NO: 33); R:5'-GAGATAATGCTTGTCTGACTTC-3' (SEQ ID NO: 34));
(F: 5'-CTTGGGGACTGAACCATCTT-3' SEQ ID NO : 3 ; R: 5'-AGCTACCATAGGGCTGGAGG-3' SEQ ID NO : 4),
(F: 5'-AAAGGCCAATGGTATATCCC-3' SEQ ID NO : 7 ; R: 5'-GCCCAGGTGATTGATAGTGC-3' SEQ ID NO : 8),
(F: 5'- CCTTGGGCCAATAAGGTAAG-3' (SEQ ID NO: 31); R: 5'-AGCTACTTGCAGTGTAACAGCATTT-3' (SEQ ID NO: 32)) ;
(F: 5'- CTGATTCATAGCAGCACTTG -3' (SEQ ID NO: 35); R:5'-AAAACATTTCCATTACCACTG-3' (SEQ ID NO: 36)).

En d'autres termes, dans ce cas de figure, les amorces utilisées permettent à la fois de démontrer l'origine foetale de la cellule testée et de détecter la mucoviscidose, en identifiant la présence ou l'absence, d'au moins un allèle portant un polymorphisme génétique, formant un linkage avec l'allèle morbide de la mucoviscidose.

D'autres amorces que celles qui précèdent peuvent être définies et utilisées pour identifier des marqueurs recherchés à l'étape c. de la méthode d'invention.

En particulier, dans un autre mode de réalisation de la méthode de détection indirecte selon l'invention, pour notamment contourner le risque qu'un des deux allèles du gène portant le marqueur recherché ne soit pas amplifié ou pas détecté (risque *d'Allele Drop Out* ou ADO), les amorces utilisées pour réaliser l'amplification à l'étape c. sont choisies pour amplifier des séquences d'ADN en dehors du chromosome 7. Ces amorces sont conçues pour être informatives selon la description donnée ci-dessus afin de permettre de discriminer des allèles paternels et des allèles maternels dans des cellules foetales. Ensuite, pour la réalisation de l'étape d., les amorces choisies pour l'amplification peuvent être des amorces identifiées à partir de l'ADN du chromosome 7, dans le cadre de ce qui est décrit dans la présente demande.

Pour définir les amorces informatives pour le génotypage de la cellule testée on choisit des oligonucléotides capables d'amplifier, par exemple par PCR, des séquences comportant des marqueurs de polymorphisme génétique distincts dans les cellules du père et de la mère : les produits d'amplification révélés sont représentés par des pics du gène concerné dans l'ADN de la cellule foetale distincts pour l'allèle paternel et pour l'allèle maternel.

De préférence, les oligonucléotides sont éloignés sur la séquence à amplifier de telle façon que la taille du produit d'amplification comporte moins de 1000, voir moins de 800 pb. La limitation de la taille de la séquence amplifiée tient compte du fait que l'ADN de la cellule testée, lorsque cette cellule est unique, est faible en quantité et est obtenue à partir d'une cellule fixée donc peut être altéré. Les amorces choisies doivent permettre l'amplification de faibles quantités d'ADN, par exemple de quantités inférieures à 5 pg, notamment d'environ 2 pg.

Dans le cadre de la méthode de détection de l'état de porteur sain ou de porteur malade de la mucoviscidose selon l'invention pour la réalisation de l'étape d. des amorces capables d'amplifier le locus deltaF508 peuvent aussi, ou alternativement être utilisées pour rechercher l'allèle deltaF508 caractéristique des mutations les plus fréquentes du gène CFTR dans les cas de mucoviscidose. Ces amorces capables d'amplifier le locus deltaF508 sont :
- le couple d'amorces externes DeltaF508 out (F : 5'-TGGAGCCTTCAGAGGGTAAA-3' SEQ ID NO :25 ; R: 5'-TGCATAATCAAAAAGTTTTCACA-3' SEQ ID NO : 26), utilisé lors de la première phase d'amplification de l'étape d), et
- le couple d'amorces internes deltaF508 in (F : 5'-TCTGTTCTCAGTTTTCCTGG-3' SEQ ID NO : 27 ; R: 5'-TCTTACCTCTTCTAGTTGGC3' SEQ ID NO : 28) utilisé lors de la deuxième phase d'amplification de l'étape d).

De façon générale, des amorces utilisables pour réaliser l'invention peuvent être constituées d'oligonucléotides variants dérivés des polynucléotides dont on a donné les séquences ci-dessus.

Un oligonucléotide variant présente par exemple au moins 60 % d'identité, de préférence 80 %, de manière préférée 95 % d'identité ou plus, avec l'amorce dont sa séquence dérive.

Ledit oligonucléotide variant présente une ou plusieurs modifications de séquence par rapport à l'amorce dont sa séquence dérive, comme en particulier une ou plusieurs modifications du type délétion, addition ou substitution.

Dans un exemple spécifique, ledit oligonucléotide variant possède la même taille que l'amorce dont sa séquence dérive.

Dans un autre exemple spécifique, la longueur dudit oligonucléotide variant possède une taille inférieure à celle de l'amorce dont sa séquence dérive, par exemple 10 à 20 % plus courte, ou éventuellement, une taille supérieure, par exemple de 10 à 40 %, en particulier de 30 à 40 % plus longue que la séquence de l'amorce dont il dérive.

Des amorces variantes des oligonucléotides cités ci-dessus peuvent par exemple être dérivées de ces oligonucléotides par addition d'un ou plusieurs nucléotides en 5' des susdites séquences.

Ledit oligonucléotide possède les mêmes propriétés que l'amorce dont il dérive s'il est utilisable comme amorce pour amplifier l'ADN d'un échantillon biologique, en particulier pour amplifier des séquences comprenant des marqueurs de l'origine foetale d'une cellule, ou pour amplifier des séquences comprenant des marqueurs de polymorphisme génétique formant un linkage avec le gène CFTR altéré caractéristique de la mucoviscidose.

Le cas échéant les oligonucléotides utilisables dans le cadre de l'invention peuvent être associés à des marqueurs, par exemple ajoutés en 5' ou en 3' de leur séquence.

Dans le cadre de la méthode de détection de l'état de porteur sain ou de porteur malade de la mucoviscidose selon l'invention, lorsqu'il n'y a pas d'enfant affecté par la mucoviscidose dans la même fratrie, les couples d'amorces utilisés en priorité au cours de l'étape d. seront des amorces capables d'amplifier le locus deltaF508 pour rechercher l'allèle deltaF508 ou d'autres mutations connues détectables directement par la méthode selon l'invention.

Dans un mode particulier de l'invention, préalablement à la démonstration de l'origine foetale ou maternelle d'une cellule collectée, ladite cellule collectée est lysée et l'ensemble de son génome est pré-amplifié, par exemple à l'aide d'amorces génériques couvrant toutes les séquences possibles selon les méthodes connues de préamplification par extension d'amorces (PEP, Primer Extension Preamplification) (Zhang, L., Cui, X., Schmitt, K., Hubert, R.WN., Arnheim, M. Whole genomic amplification from a single cell : implications for genetic analysis. PNAS 1992 89 : 5847-5851) ou encore la méthode DOP-PCR. Ces méthodes permettent d'amplifier la totalité du génome d'une seule cellule. La préparation d'ADN pré-amplifié ainsi obtenue et dérivée de l'ADN d'une cellule unique peut alors être purifiée et utilisée en tant que matériel génétique pour la détection spécifique de marqueurs génétiques ou de polymorphisme dans le cadre des étapes c) et/ou d) de la méthode selon l'invention.

Dans un exemple spécifique de l'invention, il est procédé à la démonstration de l'origine foetale ou maternelle d'une cellule collectée par l'amplification de marqueurs génétiques ou de polymorphisme ou d'une combinaison de ces marqueurs, à partir de la préparation d'ADN pré-amplifié dérivée de l'ADN d'une cellule unique. Les marqueurs de polymorphismes génétiques capables de mettre en évidence la contribution bi-parentale à l'ADN foetal sont identifiés par l'analyse préalable de l'ADN paternel et maternel au moyen d'amorces spécifiquement identifiées pour caractériser l'ADN paternel et pour caractériser l'ADN maternel ou, lorsque des amorces consensuelles existent pour déterminer l'origine paternelle ou maternelle d'un allèle d'un gène, après vérification que les amorces en question sont pertinentes pour l'analyse des échantillons contrôlés disponibles.

Dans le cadre de la présente invention, toute technique permettant l'amplification spécifique d'un acide nucléique donné peut être utilisée. Citons à titre d'exemple la méthode d'amplification par PCR *(Polymerase Chain Reaction)* ou les méthodes d'amplification isotherme telles que la TMA *(transcription mediated amplification),* la NASBA *(nucleic acid sequence based amplification),* la 3SR *(self sustained sequence replication)* ou l'amplification par déplacement de brin *(strand displacement amplification).*

Les méthodes d'amplification et en particulier, la méthode PCR, sont suffisamment sensibles pour être mises en oeuvre à partir de moins d'un cinquième de la préparation d'ADN pré-amplifié. Par conséquent, chaque préparation d'ADN pré-amplifié d'une cellule collectée peut être utilisée pour l'amplification de cinq marqueurs différents au moins.

En particulier, les amplifications réalisées par PCR permettent la détection de marqueurs de polymorphismes génétiques (par exemple 1, 2, 3, 4 ou 5 marqueurs), pour la démonstration de l'origine foetale de cellules isolées. L'amplification PCR permet aussi de détecter des marqueurs génétiques situés en amont, en aval ou sur le gène CFTR caractéristique de la mucoviscidose. Les séquences susceptibles de porter la mutation, et en particulier la délétion ou la répétition d'une séquence d'ADN, sont amplifiées et les produits d'amplification sont séparés selon leur taille, par exemple par électrophorèse. La présence de délétions ou au contraire de répétitions est détectée par la présence d'un produit d'amplification de taille inférieure ou respectivement supérieure à celle des produits d'amplification ne portant pas de délétion ou de répétition, qui peut être représentée par une différence dans la taille des pics correspondant aux dits produits d'amplification.

Les produits d'amplification peuvent aussi être séquencés, en particulier pour caractériser de manière précise les marqueurs génétiques, ou pour mettre en évidence des mutations ponctuelles.

Dans un autre mode de mise en oeuvre de la méthode de l'invention, les étapes c. et d. sont réalisées par l'hybridation de tout ou partie de la préparation d'ADN pré-amplifié ou amplifié avec des sondes d'ADN spécifiques. Les sondes d'ADN sont choisies de manière à s'hybrider spécifiquement sur les marqueurs pour leur identification ou sur les séquences portant les marqueurs à rechercher. L'hybridation des sondes sur les marqueurs peut être détectée selon les techniques classiques de détection des complexes d'hybridation d'acides nucléiques de type slot blot, Southern blot ou avantageusement aujourd'hui par des puces à ADN ou micro-réseaux ou macro-réseaux (Sambrook et al. Molecular Cloning : A laboratory Manual. 2001 3rd Ed., Cold Spring Harbor Laboratory Press, Cold Spring Harbor, New York).

Ainsi, dans un mode de réalisation de l'invention, les sondes d'ADN spécifiques des marqueurs à identifier sont fixées sur un support formant une puce à ADN ou micro-réseaux ou macro-réseaux. La préparation d'ADN pré-amplifié ou amplifié est par exemple marquée à l'aide d'un marqueur radioactif ou fluorescent, et mise en contact avec la puce à ADN ou micro-réseaux ou macro-réseaux comportant les sondes spécifiques. L'intensité d'hybridation est mesurée pour chaque spot contenant une sonde spécifique, déterminant ainsi avec une grande sensibilité la présence des marqueurs recherchés sur l'ADN d'une cellule collectée.

Lorsque la méthode de détection de l'état sain normal, de l'état de porteur sain ou de porteur malade de la mucoviscidose selon l'invention est réalisée de manière prénatale, un échantillon est prélevé chez la femme enceinte. Dans un mode de réalisation particulier de l'invention, un échantillon maternel est prélevé précocement au cours de la grossesse (par exemple autour de la cinquième semaine de grossesse). Cependant, le prélèvement de l'échantillon maternel et la méthode de diagnostic selon l'invention peuvent être réalisés à tout moment, du début jusqu'à la fin de la grossesse. Dans un mode de réalisation particulier de l'invention, le prélèvement et la méthode de diagnostic sont réalisés entre la 7^{ème} et la 15^{ème} semaine de grossesse. Dans un autre mode de réalisation, le prélèvement et la méthode de diagnostic sont réalisés entre la 10^{ème} et la 15^{ème} semaine de grossesse.

Dans le cadre d'un prélèvement de sang maternel, on prélèvera généralement entre 3 et 20 millilitres de sang maternel, de préférence entre 5 et 10 millilitres. Si, pour des raisons pratiques, on prélève entre 3 et 20 millilitres de sang maternel, il faut bien comprendre que l'analyse ultérieure des cellules foetales isolées décrite dans le cadre de l'invention, peut être réalisée sur un volume d'échantillon de sang prélevé plus limité, par exemple compris entre 1 et 10 millilitres, de préférence entre 2 et 5 millilitres. Pour augmenter la sensibilité du diagnostic, il est possible de réaliser plusieurs prélèvements indépendants afin de répéter le diagnostic sur différents échantillons indépendants. En outre, dans un mode de réalisation préféré de l'invention, il est possible de prélever en parallèle un échantillon chez le père, et un échantillon de cellules chez la mère, par exemple par prélèvement de cellules buccales par « scraping » ou par prélèvement de sang, éventuellement avant la grossesse, et de réaliser à partir du matériel prélevé, une recherche de marqueurs spécifiques du génome paternel et du génome maternel. Cette étude parallèle permet d'identifier des marqueurs génétiques spécifiques du père et de la mère, qui pourront être utilisés pour démontrer l'origine foetale des cellules isolées selon les modes de réalisation décrits dans la présente demande.

Dans le cadre de l'étape a) d'enrichissement en cellules foetales d'un échantillon maternel prélevé, pur ou dilué, susceptible de comprendre des cellules d'origine foetale, toute technique peut être employée, et notamment : la filtration ; la séparation sur gradient ; la sélection sur base immunologique (positive, les cellules marquées immunologiquement sont les cellules d'intérêt, c'est-à-dire les cellules foetales ; ou négative, les cellules marquées immunologiquement sont les cellules non pertinentes) ; la prolifération des cellules d'intérêt, c'est-à-dire les cellules foetales ; la lyse des cellules non pertinentes.

Les cellules foetales épithéliales (trophoblastiques) circulantes dans le sang ont un diamètre supérieur aux cellules leucocytaires et érythrocytaires maternelles et peuvent être isolées selon des procédés de filtration adaptés de ceux décrits pour l'isolement de cellules pathogéniques circulantes dans le sang tels que ceux décrits dans la demande de brevet FR 2782730. L'enseignement de cette demande de brevet est incorporé par référence dans la présente demande.

Dans un exemple spécifique, l'étape a) consiste en la filtration d'un échantillon maternel prélevé, pur ou dilué, susceptible de comprendre des cellules d'origine foetale, de façon à concentrer sur un filtre, selon leur taille, certaines cellules y compris des cellules d'origine foetale et, l'étape b) consiste en l'analyse des cellules retenues sur le filtre et la sélection des cellules présumées d'origine foetale.

Lorsque l'échantillon cellulaire provient du sang maternel, la filtration permet d'enrichir et de séparer les cellules foetales des cellules sanguines, en particulier des leucocytes maternels.

Préalablement à l'étape (a) de filtration, tout procédé permettant l'enrichissement de la population cellulaire issue de l'échantillon prélevé, en cellules d'origine foetale peut être mis en oeuvre. Dans une forme de réalisation de l'invention, un enrichissement de la population en cellules foetales est réalisé par le tri des cellules en fonction de l'expression de marqueurs de surfaces exprimés par les cellules foetales pour diminuer la proportion de cellules d'origine maternelle. Les techniques de tri des cellules sont par exemple le FACS, le tri sur colonne d'immunoaffinité ou immunomagnétique (MACS) ou toute technique susceptible de fournir un enrichissement d'un type cellulaire sur la base de caractéristiques physiques (densité) ou structurelles (antigènes spécifiques notamment).

Afin de faciliter la filtration, dans un mode de mise en oeuvre particulier, avant l'étape de filtration, une dilution de l'échantillon prélevé dans une solution de filtration peut être réalisée, ladite solution de filtration consistant en un réactif de fixation des cellules nucléées et/ou de lyse des globules rouges. Un exemple de solution de filtration comprend un détergent capable de dégrader la membrane des globules rouges, tel que la saponine, et un fixateur capable de stabiliser la membrane des cellules nucléées tel que le formaldéhyde.

Dans un mode de mise en oeuvre préféré, l'échantillon maternel prélevé est dilué de 10 à 100 fois environ dans la solution de filtration.

La filtration de l'échantillon pur ou dilué est réalisée à l'aide d'un filtre poreux permettant de séparer les cellules selon leur taille. Dans le cadre d'un échantillon provenant du sang maternel, la porosité du filtre est choisie de manière à laisser passer les éléments figurés du sang, notamment les érythrocytes, les plaquettes et les leucocytes maternels et à retenir certaines cellules nucléées et en particulier des cellules larges (épithéliales ou précurseurs hématopoïétiques) d'origine maternelle et foetale.

Un filtre de porosité, comprise entre 6 et 15 µm et d'une densité adaptée selon la porosité choisie et apte à retenir les cellules tout en évitant le colmatage de celles-ci lors de la filtration, peut être notamment utilisé. Selon un exemple spécifique, le filtre présente des pores sensiblement cylindriques d'un diamètre d'environ 8 µm et de densité comprise entre 5.10⁴ et 5.10⁵ pores/cm². Selon un exemple spécifique, le filtre utilisé est calibré de façon à ce que l'ensemble des pores présente un diamètre sensiblement identique. Un exemple de filtre utilisable dans le procédé de l'invention est une membrane filtrante calibrée de polycarbonate de type « Track-Etched membrane » d'une densité de pores de 1x10⁵ pores/cm², d'une épaisseur de 12µm et d'une taille des pores de 8µm, telle que celle commercialisée par Whatman®.

L'étape a. de la méthode selon l'invention repose sur l'existence et la mise au point d'un dispositif spécifique nommé ISET (acronyme anglais pour Isolation by Size of Epithelial Tumor Cells) et décrit dans EP 0513 139 et comprenant sur un bâti:
- un filtre poreux apte à retenir certaines cellules (circulantes dans le cadre d'un prélèvement de sang maternel) selon leur taille, monté entre deux dispositifs de serrage, respectivement en amont et en aval du sens de filtration, et destiné à l'étanchéité de la filtration,
- le bloc amont comprenant des moyens de stockage et/ou de pré-traitement des échantillons à analyser,
- le bloc aval comprenant des perforations en regard des moyens de stockage pour collecter les déchets,
- des moyens de filtration forcée.

Ainsi, l'invention porte également sur l'adaptation et l'utilisation d'un dispositif de ce type à la filtration de cellules foetales présentes dans l'échantillon maternel prélevé pour les besoins de la méthode de détection, non invasive, prénatale de la mucoviscidose.

Par adaptation et utilisation, on entend:
- l'incorporation dans le dispositif d'un filtre de porosité, de préférence calibré, apte à retenir les cellules d'un diamètre moyen supérieur à 8 µm, et mieux, supérieur à 10 µm, de préférence supérieur à 15 µm. Un filtre de porosité moyenne de 8 µm a été démontré comme répondant à ces caractéristiques recherchées ;
- l'adaptation de la densité des pores sur le filtre, en fonction de la taille des pores afin d'optimiser la capacité de filtration,
- l'adaptation de la pression appliquée sur les moyens de filtration à la conservation de l'intégrité physique des cellules foetales recherchées ;
- l'adaptation du milieu de dilution de l'échantillon maternel prélevé à la conservation de l'intégrité et de la viabilité des cellules recherchées.

L'étape a. de la méthode selon l'invention peut comporter l'utilisation d'un dispositif de filtration de type ISET, pour l'isolement de cellules foetales de l'échantillon cellulaire maternel prélevé et comportant un filtre de porosité moyenne comprise entre 6µm et 15 µm, et de préférence d'environ 8 µm.

L'étape a. peut comporter également l'utilisation d'un dispositif de type ISET dans lequel le filtre présente des pores d'un diamètre d'environ 8 µm et une densité de pores comprise entre 5.10⁴ et 5.10⁵.

L'étape a. peut comporter enfin l'utilisation d'un dispositif de filtration de type ISET dans lequel la dépression de filtration appliquée est comprise entre 0,05 bars et 0,8 bars, et de préférence environ 0,1 bars.

Dans un exemple spécifique de l'invention, des cellules retenues sur le filtre pendant l'étape a. sont collectées individuellement.

Par collecte individuelle des cellules, il faut comprendre toute méthode qui permet de collecter une cellule individuelle spécifique retenue pendant l'étape a) en vue de son analyse ultérieure indépendamment des autres cellules retenues pendant l'étape a).

Plus particulièrement, des cellules retenues pendant l'étape a) peuvent être collectées individuellement notamment par microdissection.

Dans un exemple spécifique, l'étape a) consiste en une filtration, et la microdissection consiste par exemple à découper au laser la partie d'une membrane filtrante sur laquelle est retenue une cellule ou à décoller la cellule à l'aide du laser puis à récupérer la cellule unique collectée dans un tube approprié. Celle-ci est alors apte à subir les différentes analyses des étapes b), c) et d) décrites dans le cadre de l'invention.

Dans un exemple spécifique, les cellules retenues pendant l'étape a) peuvent être analysées « in situ » au cours de l'étape b), sans collecte individuelle des cellules. Cette analyse est effectuée par une méthode cytologique, immunologique ou moléculaire, par exemple une méthode parmi les suivantes : FISH, PCR in situ, PNA, PRINS.

Les cellules retenues pendant l'étape a) sont collectés, puis analysées in situ au cours de l'étape b).

La collecte individuelle des cellules permet de cibler avantageusement l'analyse génétique sur le génome d'une cellule unique. Elle permet aussi de réaliser la détection de la mucoviscidose sur le génome d'une cellule unique dont l'origine foetale a été préalablement démontrée par analyse génétique. Ainsi, par ce mode de réalisation de l'invention, on obtient du matériel génétique pur, c'est à dire, dérivé d'une cellule unique, et utilisable à la fois pour les étapes c. et d. de la méthode selon l'invention.

Dans le cadre de la présente méthode de détection, non invasive, prénatale de la mucoviscidose, on a illustré l'utilisation de différentes amorces appropriées pour la réalisation de l'étape c. ou de l'étape d., voir des deux étapes. Selon une variante de réalisation de cette méthode, d'autres amorces peuvent être mises en oeuvre qui consistent par exemple en des oligonucléotides variants dont la séquence est dérivée de celle d'une amorce choisie parmi :
- F: 5'-AAAAACCCTGGCTTATGC-3' (SEQ ID NO : 1) ; R: 5'-AGCTACCATAGGGCTGGAGG-3' (SEQ ID NO : 2) ; ou
- F: 5'-GGAATCTGTTCTGGCAATGGAT-3' (SEQ ID NO : 5); R: 5'-TTGCAATGAGCCGAGATCCTG-3' (SEQ ID NO : 6) ; ou
- F: 5'-CAGATGCTCGTTGTGCACAA-3' (SEQ ID NO : 9); R: 5'-ATACCATTTACGTTTGTGTGTG-3' (SEQ ID NO : 10) ; ou
- F : 5'-TGACAGTGCAGCTCATGGTC-3' (SEQ ID NO : 13); R : 5'-GGTCATTGGTCAAGGGCTGCT-3' (SEQ ID NO : 14); ou
- F: 5'-TTGACATTCTTCTGTAAGGAAGA-3' (SEQ ID NO : 17); R: 5'-AGGCTTGCCAAAGATATTAAAAG-3' (SEQ ID NO : 18) ;ou
- F: 5'-TTGTGAATAGTGCTGCAATG-3' (SEQ ID NO : 21); R: 5'-ATGTACACTGACTTGTTTGAG-3' (SEQ ID NO : 22) ; ou
- F : 5'-TGGAGCCTTCAGAGGGTAAA-3' (SEQ ID NO : 25) ; R : 5'-TGCATAATCAAAAAGTTTTCACA-3' (SEQ ID NO : 26) ;ou
- F: 5'- AAGTAATTCTCCTGCCTCAG-3' (SEQ ID NO: 29); R: 5'-AGCTACTTGCAGTGTAACAGCATTT -3' (SEQ ID NO: 30) ;
- F: 5'- GAATTATAACCGTAACTGATTC-3' (SEQ ID NO: 33) ; R:5'-GAGATAATGCTTGTCTGACTTC-3' (SEQ ID NO: 34) ;
- F: 5'-CTTGGGGACTGAACCATCTT-3' (SEQ ID NO : 3); R: 5'-AGCTACCATAGGGCTGGAGG-3' (SEQ ID NO : 4) ; ou
- F: 5'-AAAGGCCAATGGTATATCCC-3' (SEQ ID NO : 7); R: 5'-GCCCAGGTGATTGATAGTGC-3' (SEQ ID NO : 8) ; ou
- F: 5'-GATCCCAAGCTCTTCCTCTT-3'(SEQ ID NO : 11); R: 5'-ACGTTTGTGTGTGCATCTGT-3' (SEQ ID NO : 12); ou
- F: 5'-GGATAAACATAGAGCGACAGTTC-3' (SEQ ID NO : 15); R: 5'-AGACAGAGTCCCAGGCATT-3' (SEQ ID NO : 16) ;ou
- F: 5'-CCCTCTCAATTGTTTGTCTACC-3' (SEQ ID NO : 19); R: 5'-GCAAGAGATTTCAGTGCCAT-3' (SEQ ID NO : 20) ; ou
- F : 5'-ATGTACATGTGTCTGGGAAGG-3' (SEQ ID NO : 23); R : 5'-TTCTCTACATATTTACTGCCAACA-3' (SEQ ID NO : 24) ;
- F : 5'-TCTGTTCTCAGTTTTCCTGG-3' (SEQ ID NO : 27) ; R : (5'-TCTTACCTCTTCTAGTTGGC-3' (SEQ ID NO : 28) ;
- F: 5'- CCTTGGGCCAATAAGGTAAG-3' (SEQ ID NO: 31); R: 5'-AGCTACTTGCAGTGTAACAGCATTT-3' (SEQ ID NO: 32) ;
- F: 5'- CTGATTCATAGCAGCACTTG -3' (SEQ ID NO: 35) ; R:5'-AAAACATTTCCATTACCACTG-3' (SEQ ID NO: 36).

Un oligonucléotide variant présente par exemple au moins 60 % d'identité, de préférence 80 %, de manière préférée 95 % d'identité ou plus, avec l'amorce dont sa séquence dérive.

Ledit oligonucléotide variant présente une ou plusieurs modifications de séquence par rapport à l'amorce dont sa séquence dérive, comme en particulier une ou plusieurs modifications du type délétion, addition ou substitution. Les nucléotides ajoutés le sont en particulier à l'extrémité 3'.

Dans un exemple spécifique, ledit oligonucléotide variant possède la même taille que l'amorce dont sa séquence dérive.

Dans un autre exemple spécifique, la longueur dudit oligonucléotide variant possède une taille inférieure, par exemple de 10 à 20 % par rapport à celle de l'amorce dont sa séquence dérive ou éventuellement, une taille supérieure, par exemple de 10 à 40 %, en particulier de 30 à 40 % par rapport à la séquence de l'amorce dont il dérive.

Ledit oligonucléotide possède les mêmes propriétés que l'amorce dont il dérive s'il est utilisable comme amorce pour amplifier l'ADN d'un échantillon cellulaire biologique, en particulier pour amplifier des séquences comprenant des marqueurs de l'origine foetale d'une cellule, ou pour amplifier des séquences comprenant des marqueurs génétiques situés en amont, en aval ou sur le gène CFTR caractéristique de la mucoviscidose.

Au cours de l'étape b. de la méthode selon l'invention, les cellules retenues sur le filtre peuvent être observées sous microscope, par exemple après coloration à l'hématoxyline et l'éosine de manière à analyser leur morphologie dans le but de déterminer une présomption de leur origine foetale. Leur nature épithéliale peut être identifiée, à titre d'exemple, par immunomarquage avec un anticorps anti-cytokeratines (type KL₁). Il est à ce stade possible d'envisager de reconnaître sur la base de caractères morphologiques les cellules d'origine foetale et en particulier, les cellules cytotrophoblastiques, mononucléées, présentant un grand noyau, une chromatine condensée et un cytoplasme réduit, d'un diamètre compris entre 14 et 20 µm et les cellules syncytiotrophoblastiques d'un plus grand diamètre (44-47 µm ou plus) et plurinucléées.

Le terme « présomption » ou « présumée » à l'étape (b) indique donc la forte probabilité d'être en présence d'une cellule d'origine foetale.

L'analyse génétique des cellules retenues pendant l'étape a) permet de conforter la présomption, par l'obtention des indications sur l'origine foetale ou maternelle de chacune de ces cellules. En particulier, en vue d'une détection ou d'un diagnostic sensible et spécifique, l'étape a) sera répétée si aucune cellule présumée d'origine foetale n'est observée suite à celle-ci. Selon les modes de réalisation, l'analyse génétique sera établie sur l'ensemble des cellules retenues pendant l'étape a) et comprenant certaines cellules dont l'origine foetale est présumée, en particulier sur le génome d'un petit nombre de cellules, allant de 1 à 20 cellules, plus particulière de 1 à 10 cellules, ou au contraire l'analyse sera réalisée sur, par exemple, le(s) génome(s) d'1, 2 ou 3 cellules et avantageusement sur le génome de cellules isolées individuellement.

Dans un mode de mise en oeuvre particulier, on procède à l'analyse de présomption de l'origine foetale ou maternelle des cellules retenues sur le filtre par la recherche de la présence de marqueur(s) immunologique(s) ou cytologique(s) caractéristique(s) des cellules foetales.

On entend par marqueur immunologique caractéristique des cellules foetales tout antigène ou combinaison d'antigènes dont l'expression est normalement significativement différente entre les cellules foetales et les cellules maternelles, et qui peut être détectée à l'aide d'un anticorps ou d'une combinaison d'anticorps dirigés spécifiquement contre ledit antigène ou combinaison d'antigènes. Des exemples de tels marqueurs immunologiques sont en particulier les antigènes associés aux cellules trophoblastiques décrits dans la demande de brevet WO 90/06509. L'enseignement de cette demande de brevet est incorporé par référence dans la présente demande. La recherche de la présence de marqueurs immunologiques caractéristiques des cellules foetales consiste par exemple :
- en la mise en contact des cellules contenues dans l'échantillon maternel prélevé avec au moins un anticorps dirigé contre un antigène caractéristique des cellules foetales ; et,
- la détermination sur les cellules retenues sur le filtre d'une liaison spécifique dudit anticorps avec un antigène exprimé à la surface desdites cellules, Ladite mise en contact des cellules avec l'anticorps peut être réalisée avant ou après l'étape de filtration. Les anticorps choisis peuvent être du type polyclonal ou monoclonal.

Un exemple d'antigène caractéristique des cellules foetales est l'antigène de la phosphatase alcaline placentaire.

Dans un autre mode de mise en oeuvre, on peut procéder à l'analyse de présomption de l'origine foetale des cellules par la détermination de marqueurs cytologiques spécifiques des cellules cytotrophoblastiques et/ou syncyciotrophoblastiques. Les marqueurs cytologiques utilisables comprennent l'ensemble des caractéristiques cytologiques des cellules foetales permettant de les différencier des autres types de cellules circulantes susceptibles d'être retenues sur le filtre, en particulier, la taille des cellules, la forme des cellules, la présence et la taille d'organites particuliers, la taille et le nombre de noyau, la structure de la chromatine, etc., ou toutes combinaisons particulières de ces caractéristiques cytologiques. Les caractères cytologiques peuvent être observés par coloration des cellules à l'aide de colorants classiquement utilisés en cytologie, en particulier l'hématoxyline-éosine et par l'observation des cellules marquées en microscopie optique.

L'objet de l'invention porte également sur un polynucléotide utilisable comme amorce pour amplifier une quantité d'ADN d'un échantillon biologique caractérisé en ce qu'il comprend ou consiste en une séquence de nucléotides choisie parmi les suivants:
- F: 5'-AAAAACCCTGGCTTATGC-3' ; R: 5'-AGCTACCATAGGGCTGGAGG-3' ; ou
- F : 5'-GGAATCTGTTCTGGCAATGGAT-3' ; R : 5'-TTGCAATGAGCCGAGATCCTG-3' ; ou
- F: 5'-CAGATGCTCGTTGTGCACAA-3'; R: 5'-ATACCATTTACGTTTGTGTGTG-3', ou
- F: 5'-TGACAGTGCAGCTCATGGTC-3' ; R : 5'-GGTCATTGGTCAAGGGCTGCT-3' ; ou
- F: 5'-TTGACATTCTTCTGTAAGGAAGA-3' ; R: 5'-AGGCTTGCCAAAGATATTAAAAG-3' ; ou
- F: 5'-TTGTGAATAGTGCTGCAATG-3'; R: 5'-ATGTACACTGACTTGTTTGAG-3' ; ou
- F : 5'-TGGAGCCTTCAGAGGGTAAA-3'; R: 5'-TGCATAATCAAAAAGTTTTCACA-3' ; ou
- F : 5'-TCTGTTCTCAGTTTTCCTGG-3' ; R: (5'-TCTTACCTCTTCTAGTTGGC-3' ; ou
- F: 5'- AAGTAATTCTCCTGCCTCAG-3' (SEQ ID NO: 29); R: 5'-AGCTACTTGCAGTGTAACAGCATTT -3' (SEQ ID NO: 30) ;
- F: 5'- GAATTATAACCGTAACTGATTC-3' (SEQ ID NO: 33) ; R:5'-GAGATAATGCTTGTCTGACTTC-3' (SEQ ID NO: 34).

Un oligonucléotide variant présente par exemple au moins 60 % d'identité, de préférence 80 %, de manière préférée 95 % d'identité ou plus, avec l'amorce dont sa séquence dérive.

Ledit oligonucléotide variant présente une ou plusieurs modifications de séquence par rapport à l'amorce dont sa séquence dérive, comme en particulier une ou plusieurs modifications du type délétion, addition ou substitution.

Dans un exemple spécifique, ledit oligonucléotide variant possède la même taille que l'amorce dont sa séquence dérive.

Dans un autre exemple spécifique, la longueur dudit oligonucléotide variant possède une taille inférieure de 10 à 20 % par rapport à celle de l'amorce dont sa séquence dérive ou éventuellement, une taille supérieure, par exemple de 10 à 40 %, en particulier de 30 à 40 % par rapport à la séquence de l'amorce dont il dérive.

Des amorces variantes des oligonucléotides cités ci-dessus peuvent par exemple être dérivées de ces oligonucléotides par addition d'un ou plusieurs nucléotides en 5' des susdites séquences.

Ledit oligonucléotide possède les mêmes propriétés que l'amorce dont il dérive s'il est utilisable comme amorce pour amplifier l'ADN d'un échantillon cellulaire biologique, en particulier pour amplifier des séquences comprenant des marqueurs de l'origine foetale d'une cellule, ou pour amplifier des séquences comprenant une mutation du locus F508 ou des séquences comprenant des marqueurs de polymorphisme génétique formant un linkage avec le gène CFTR caractéristique de la mucoviscidose.

L'objet de l'invention porte également sur un couple de polynucléotides, utilisable comme couple d'amorces pour amplifier une quantité d'ADN d'un échantillon biologique, caractérisé en ce qu'il est choisi parmi les couples de polynucléotides dont les séquences comprennent ou consistent en:
- F : 5'-AAAAACCCTGGCTTATGC-3' ; R : 5'-AGCTACCATAGGGCTGGAGG-3' ;
- F: 5'-GGAATCTGTTCTGGCAATGGAT-3' ; R : 5'-TTGCAATGAGCCGAGATCCTG-3' ;
- F : 5'-CAGATGCTCGTTGTGCACAA-3'; R: 5'-ATACCATTTACGTTTGTGTGTG-3';
- F : 5'-TGACAGTGCAGCTCATGGTC-3', R: 5'-GGTCATTGGTCAAGGGCTGCT-3' ;,
- F: 5'-TTGACATTCTTCTGTAAGGAAGA-3'; R: 5'-AGGCTTGCCAAAGATATTAAAAG-3' ;
- F : 5'-TTGTGAATAGTGCTGCAATG-3' ; R : 5'-ATGTACACTGACTTGTTTGAG-3' ;
- F : 5'-TGGAGCCTTCAGAGGGTAAA-3' ; R: 5'-TGCATAATCAAAAAGTTTTCACA-3' ;
- F : 5'-TCTGTTCTCAGTTTTCCTGG-3'; R: (5'-TCTTACCTCTTCTAGTTGGC-3' ;
- F: 5'- AAGTAATTCTCCTGCCTCAG-3' (SEQ ID NO: 29); R: 5'-AGCTACTTGCAGTGTAACAGCATTT -3' (SEQ ID NO: 30) ;
- F: 5'- GAATTATAACCGTAACTGATTC-3' (SEQ ID NO: 33) ; R:5'-GAGATAATGCTTGTCTGACTTC-3' (SEQ ID NO: 34).

L'objet de l'invention porte également sur l'association d'un couple d'amorces dites externes, par rapport à la séquence cible, utilisé dans une première phase d'amplification d'une préparation d'ADN et d'un autre couple d'amorces, dites internes ou emboîtées par rapport aux dites amorces externes, utilisé dans une deuxième phase d'amplification sur le produit d'amplification obtenu par ladite première phase d'amplification, lesdits couples étant choisis parmi:
- F : 5'-AAAAACCCTGGCTTATGC-3' ; R: 5'-AGCTACCATAGGGCTGGAGG-3' en tant qu'amorces externes et F: 5'-CTTGGGGACTGAACCATCTT-3' ; R : 5'-AGCTACCATAGGGCTGGAGG-3' en tant qu'amorces internes; ou
- F: 5'-GGAATCTGTTCTGGCAATGGAT-3'; R: 5'-TTGCAATGAGCCGAGATCCTG-3' en tant qu'amorces externes et -F : 5'-AAAGGCCAATGGTATATCCC-3' ; R : 5'-GCCCAGGTGATTGATAGTGC-3' en tant qu'amorces internes; ou
- F: 5'-CAGATGCTCGTTGTGCACAA-3'; R: 5'-ATACCATTTACGTTTGTGTGTG-3' en tant qu'amorces externes et -F : 5'-GATCCCAAGCTCTTCCTCTT-3' ; R : 5'-ACGTTTGTGTGTGCATCTGT-3' en tant qu'amorces internes; ou
- F: 5'-TGACAGTGCAGCTCATGGTC-3' R: 5'-GGTCATTGGTCAAGGGCTGCT-3' ; en tant qu'amorces externes et -F : 5'-GGATAAACATAGAGCGACAGTTC-3' ; R: 5'-AGACAGAGTCCCAGGCATT-3' en tant qu'amorces internes ; ou
- F : 5'-TTGACATTCTTCTGTAAGGAAGA-3' ; R : 5'-AGGCTTGCCAAAGATATTAAAAG-3' en tant qu'amorces externes et -F : 5'-CCCTCTCAATTGTTTGTCTACC-3' ; R : 5'-GCAAGAGATTTCAGTGCCAT-3' en tant qu'amorces internes ; ou
- F : 5'-TTGTGAATAGTGCTGCAATG-3' ; R: 5'-ATGTACACTGACTTGTTTGAG-3' en tant qu'amorces externes et F : 5'-ATGTACATGTGTCTGGGAAGG-3' ; R : 5'-TTCTCTACATATTTACTGCCAACA-3' en tant qu'amorces internes, ou
- F : 5'-TGGAGCCTTCAGAGGGTAAA-3'; R: 5'-TGCATAATCAAAAAGTTTTCACA-3' en tant qu'amorces externes et F : 5'-TCTGTTCTCAGTTTTCCTGG-3' ; R : 5'-TCTTACCTCTTCTAGTTGGC-3' en tant qu'amorces internes ; ou
- F: 5'- AAGTAATTCTCCTGCCTCAG-3' (SEQ ID NO: 29) ; R: 5'-AGCTACTTGCAGTGTAACAGCATTT -3' (SEQ ID NO: 30) en tant qu'amorces externes et F: 5'- CCTTGGGCCAATAAGGTAAG-3' (SEQ ID NO: 31) ; R: 5'-AGCTACTTGCAGTGTAACAGCATTT-3' (SEQ ID NO: 32) en tant qu'amorces internes ; ou
- F: 5'- GAATTATAACCGTAACTGATTC-3' (SEQ ID NO: 33); R:5'-GAGATAATGCTTGTCTGACTTC-3' (SEQ ID NO: 34) en tant qu'amorces externes, et F: 5'- CTGATTCATAGCAGCACTTG -3' (SEQ ID NO: 35) ; R:5'-AAAACATTTCCATTACCACTG-3' (SEQ ID NO: 36) en tant qu'amorces internes.

L'invention concerne également l'utilisation d'amorces dans le cadre d'une méthode de détection, non invasive prénatale, *in vitro* de l'état sain normal, de l'état de porteur sain ou de l'état de porteur malade de la mucoviscidose à partir de l'ADN génomique de cellules foetales isolées de l'échantillon maternel prélevé, caractérisée en ce que les amorces sont choisies parmi:
- F: 5'-AAAAACCCTGGCTTATGC-3' ; R : 5'-AGCTACCATAGGGCTGGAGG-3' ;
- F: 5'-GGAATCTGTTCTGGCAATGGAT-3'; R: 5'-TTGCAATGAGCCGAGATCCTG-3' ;
- F : 5'-CTTGGGGACTGAACCATCTT-3' ; R: 5'-AGCTACCATAGGGCTGGAGG-3' ;
- F : 5'-AAAGGCCAATGGTATATCCC-3' ; R : 5'-GCCCAGGTGATTGATAGTGC-3' ;
- F: 5'- AAGTAATTCTCCTGCCTCAG-3' (SEQ ID NO: 29) ; R: 5'-AGCTACTTGCAGTGTAACAGCATTT -3' (SEQ ID NO: 30) ;
- F: 5'- GAATTATAACCGTAACTGATTC-3' (SEQ ID NO: 33); R:5'-GAGATAATGCTTGTCTGACTTC-3' (SEQ ID NO: 34) ;
- F: 5'- CCTTGGGCCAATAAGGTAAG-3' (SEQ ID NO: 31) ; R: 5'-AGCTACTTGCAGTGTAACAGCATTT-3' (SEQ ID NO: 32) ;
- F: 5'- CTGATTCATAGCAGCACTTG -3' (SEQ ID NO: 35) ; R:5'-AAAACATTTCCATTACCACTG-3' (SEQ ID NO: 36) ;
- F : 5'-TGGAGCCTTCAGAGGGTAAA-3' ; R : 5'-TGCATAATCAAAAAGTTTTCACA-3' ;
- F : 5'-TCTGTTCTCAGTTTTCCTGG-3' ; R : (5'-TCTTACCTCTTCTAGTTGGC-3'.

L'invention concerne également l'utilisation d'amorces dans le cadre d'une méthode *in vitro* pour l'identification du caractère foetal d'une cellule unique collectée à partir de l'échantillon maternel prélevé, sur une préparation d'ADN dérivée du génome de la cellule unique collectée, caractérisée en ce que les amorces sont choisies parmi :
- F : 5'-AAAAACCCTGGCTTATGC-3' ; R : 5'-AGCTACCATAGGGCTGGAGG-3' ;
- F: 5'-GGAATCTGTTCTGGCAATGGAT-3'; R: 5'-TTGCAATGAGCCGAGATCCTG-3' ;
- F: 5'-CAGATGCTCGTTGTGCACAA-3'; R: 5'-ATACCATTTACGTTTGTGTGTG-3' ;
- F: 5'-TGACAGTGCAGCTCATGGTC-3' ; R: 5'-GGTCATTGGTCAAGGGCTGCT-3' ; ou
- F : 5'-TTGACATTCTTCTGTAAGGAAGA-3' ; R : 5'-AGGCTTGCCAAAGATATTAAAAG-3' ;
- F : 5'-TTGTGAATAGTGCTGCAATG-3' ; R: 5'-ATGTACACTGACTTGTTTGAG-3' ;
- F: 5'- AAGTAATTCTCCTGCCTCAG-3' (SEQ ID NO: 29); R: 5'-AGCTACTTGCAGTGTAACAGCATTT -3' (SEQ ID NO: 30) ;
- F: 5'- GAATTATAACCGTAACTGATTC-3' (SEQ ID NO: 33) ; R:5'-GAGATAATGCTTGTCTGACTTC-3' (SEQ ID NO: 34) ;
- F : 5'-CTTGGGGACTGAACCATCTT-3' ; R : 5'-AGCTACCATAGGGCTGGAGG-3' ;
- F : 5'-AAAGGCCAATGGTATATCCC-3' ; R: 5'-GCCCAGGTGATTGATAGTGC-3' ;
- F : 5'-GATCCCAAGCTCTTCCTCTT-3'; R: 5'-ACGTTTGTGTGTGCATCTGT-3' ;
- F : 5'-GGATAAACATAGAGCGACAGTTC-3' ; R: 5'-AGACAGAGTCCCAGGCATT-3' ;
- F: 5'-CCCTCTCAATTGTTTGTCTACC-3' ; R: 5'-GCAAGAGATTTCAGTGCCAT-3' ;
- F: 5'-ATGTACATGTGTCTGGGAAGG-3'; R: 5'-TTCTCTACATATTTACTGCCAACA-3' ;
- F: 5'- CCTTGGGCCAATAAGGTAAG-3' (SEQ ID NO: 31); R: 5'-AGCTACTTGCAGTGTAACAGCATTT-3' (SEQ ID NO: 32) ;
- F: 5'- CTGATTCATAGCAGCACTTG -3' (SEQ ID NO: 35) ; R:5'-AAAACATTTCCATTACCACTG-3' (SEQ ID NO: 36).

L'objet de l'invention porte également sur un kit pour à la détection, non invasive, prénatale de l'état sain normal, de l'état de porteur sain ou de l'état de porteur malade de la mucoviscidose comprenant une ou plusieurs amorces citées ci-dessus, et le cas échéant des instructions pour effectuer la détection la mucoviscidose.

Les méthodes et moyens (notamment les amorces) mis en oeuvre dans le cadre de la présente invention sont utilisables dans le cadre de la détection de la mucoviscidose et peuvent le cas échéant être considérés, en tant que tels, ou complétés par d'autres tests, comme des méthodes et moyens de diagnostic.

### LEGENDE DES FIGURES

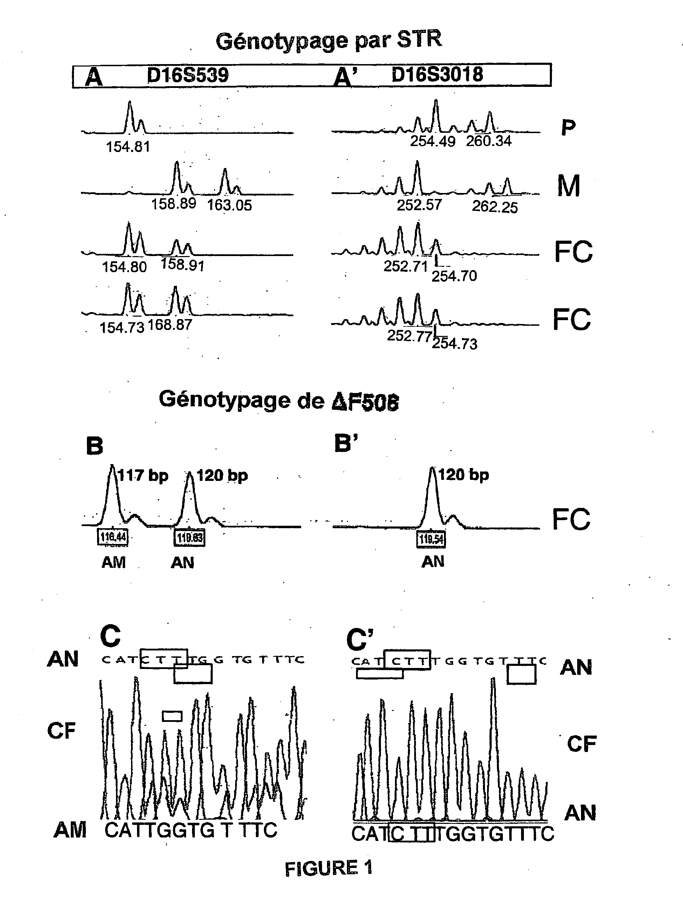

**Figure 1** **: Diagnostic prénatal non invasif chez des couples de porteurs de l'allèle deltaF508**
   Génotypage par STR chez le couple 1 (A) avec le marqueur D16S539 et le couple 6 (A') avec le marqueur D16S3018. Chez le couple 1 (A), le père (P) est homozygote pour l'allèle ciblé, tandis que la mère (M) est hétérozygote. Deux cellules foetales (CF) sont montrées, caractérisées par un allèle paternel (à gauche) et un allèle maternel (à droite). Chez le couple 6 (A'), les deux parents sont hétérozygotes avec le marqueur STR utilisé et les deux cellules foetales (CF) présentent un allèle maternel (à gauche) et un allèle paternel (à droite). Le génotypage de deltaF508 (B et B') effectué sur les cellules foetales du couple 1 (B) et 6 (B') montre que le foetus du couple 1 est porteur de l'allèle deltaF508, ayant un allèle muté (AM) et un allèle normal (AN), tandis que le foetus du couple 6 est totalement normal (homozygote pour l'allèle normal). C et C' : le séquençage du locus deltaF508 dans des cellules foetales confirme le diagnostic en montrant à la fois le profil muté et normal chez le foetus du couple 1 (C), et la présence homozygote de l'allèle normal chez le foetus du couple 6 (C').

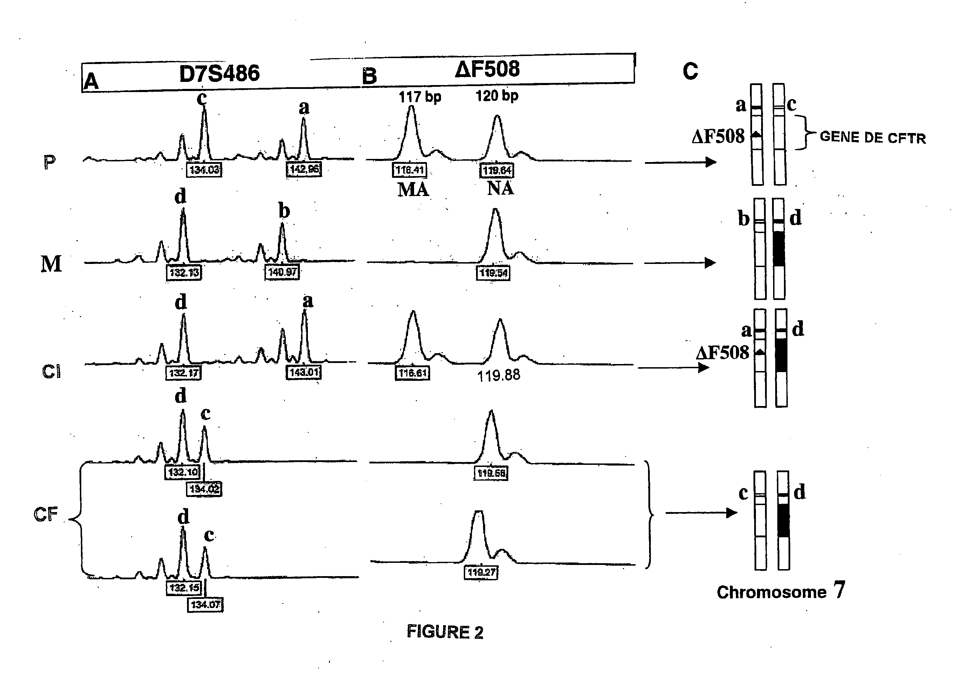

**Figure 2** **: Diagnostic prénatal non invasif chez le couple n°11**
   A. Génotypage d'ADN avec le marqueur informatif D7S486 sur le chromosome 7, montrant que les parents (P et M) sont hétérozygotes et que l'enfant affecté (CI : cas index) porte les allèles mutés d et a. Les deux cellules foetales (CF) de la grossesse en cours portent un seul allèle muté (d), démontrant que le foetus est porteur mais non affecté.
   B. Génotypage d'ADN du locus deltaF508 montrant que le père est porteur d'un allèle muté (AM) et d'un allèle normal (AN). Le cas index est porteur de l'allèle deltaF508 muté, tandis que les deux cellules foetales de la grossesse en cours sont homozygotes pour l'allèle normal. Ainsi, le foetus est porteur d'une mutation sur le chromosome 7 qui n'est pas la mutation deltaF508.
   C. Représentation schématique des deux chromosomes 7 dans l'ADN paternel (P), l'ADN maternel (M), le cas index (CI) et les cellules foetales circulantes (CF) : le père est porteur d'un allèle deltaF508, tandis que la mère est porteuse d'une mutation inconnue. Le cas index porte les deux mutations et les cellules foetales portent uniquement la mutation inconnue.

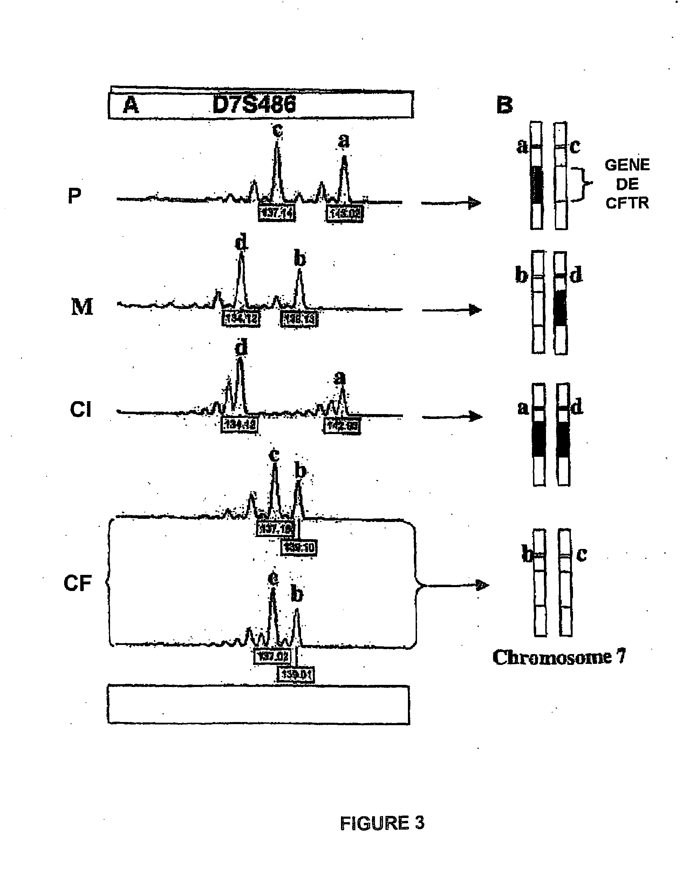

**Figure 3** **: Diagnostic prénatal non invasif chez le couple n°11**
   A. Génotypage d'ADN avec le marqueur informatif D7S486 sur le chromosome 7, montrant que les parents (P et M) sont hétérozygotes et que le cas index (CI) porte les allèles mutés d et a. Les deux cellules foetales (CF) de la grossesse en cours portent les allèles non mutés (c et b), démontrant que le foetus est totalement normal.
   B. Représentation schématique des deux chromosomes 7 dans l'ADN paternel (P), l'ADN maternel (M), le cas index (CI) et les cellules foetales circulantes (CF): le père est porteur de l'allèle muté a, tandis que la mère est porteuse de l'allèle muté d. L'enfant affecté porte les deux allèles mutés et les deux cellules foetales circulantes portent les deux allèles normaux.

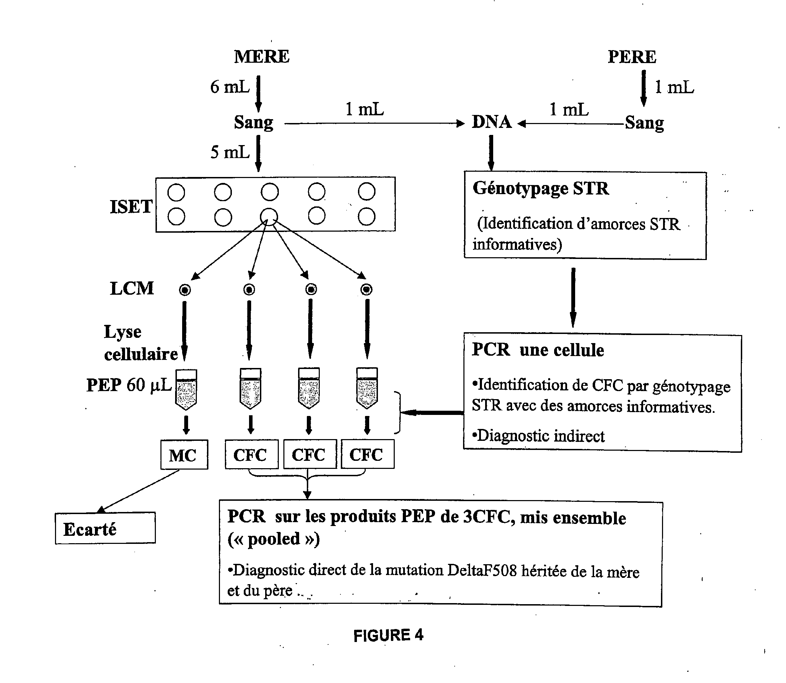

**Figure 4** **: Schéma de protocole pour le diagnostic prénatal non invasif (NI-PND) de la mucoviscidose**
   STR (short tandem repeat) courtes séquences répétées en tandem;
   ISET *(isolation by Size of Epithelial Tumor*/*Trophoblastic cells),* isolement selon la taille des cellules tumorales épithéliales/trophoblastiques ;
   LCM *(laser capture microdissection)* microdissection par capture au laser ;
   PEP *(primer extension preamplification)* pré-amplification par élongation d'amorces ;
   MC *(maternal cells)* cellules maternelles ;
   CFC *(circulating fetal cells)* cellules foetales circulantes.

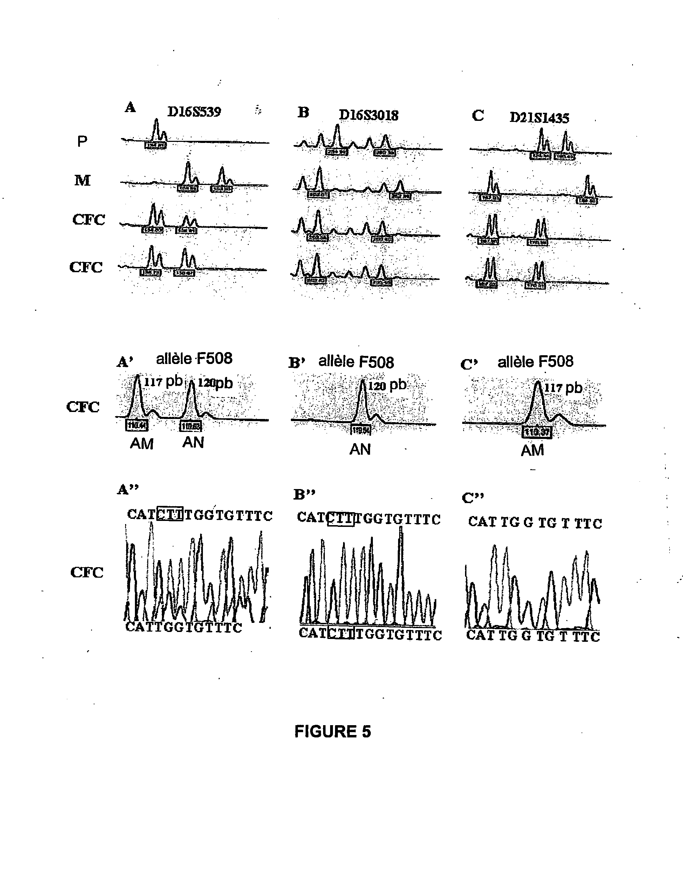

**Figure 5** **: Diagnostic prénatal non invasif chez des couples de porteurs de l'allèle deltaF508**
   A, B, C : Génotypage par STR chez le couple 1 avec le marqueur D16S539 (A), chez le couple 6 avec le marqueur D16S3018 (B) et chez le couple 8 avec le marqueur D2151435 (C).
   Chez le couple 1 (A), le père (P) est homozygote pour l'allèle ciblé (un pic), tandis que la mère (M) est hétérozygote (deux pics). Deux cellules foetales circulantes (CFC) de ce couple sont caractérisées par un allèle paternel (à gauche) et un allèle maternel (à droite). Chez le couple 6 (B), et le couple 8 (C), les deux parents sont hétérozygotes avec le marqueur STR utilisé et les deux cellules foetales (CFC) présentent un allèle maternel (à gauche) et un allèle paternel (à droite).
   A', B', C' : Le génotypage de deltaF508 effectué sur les cellules foetales circulantes (CFC) du couple 1 (A'), du couple 6 (B'), et du couple 8 (C') montre que le foetus du couple 1 est porteur de l'allèle deltaF508, ayant un allèle muté (AM) et un allèle normal (AN), tandis que le foetus du couple 6 est totalement normal (homozygote pour l'allèle normal), et le foetus du couple 8 est affecté par le mucoviscidose (CF) (homozygote pour l'allèle muté).
   A", B", C" : séquençage du locus F508 dans des cellules foetales du couple 1 (A"), du couple 6 (B") et du couple 8 (C") confirme le diagnostic en montrant à la fois le profil muté et normal chez le foetus du couple 1 (A"), et la présence homozygote de l'allèle normal chez le foetus du couple 6 (B"), et la présence homozygote de l'allèle muté chez le foetus du couple 8 (C").

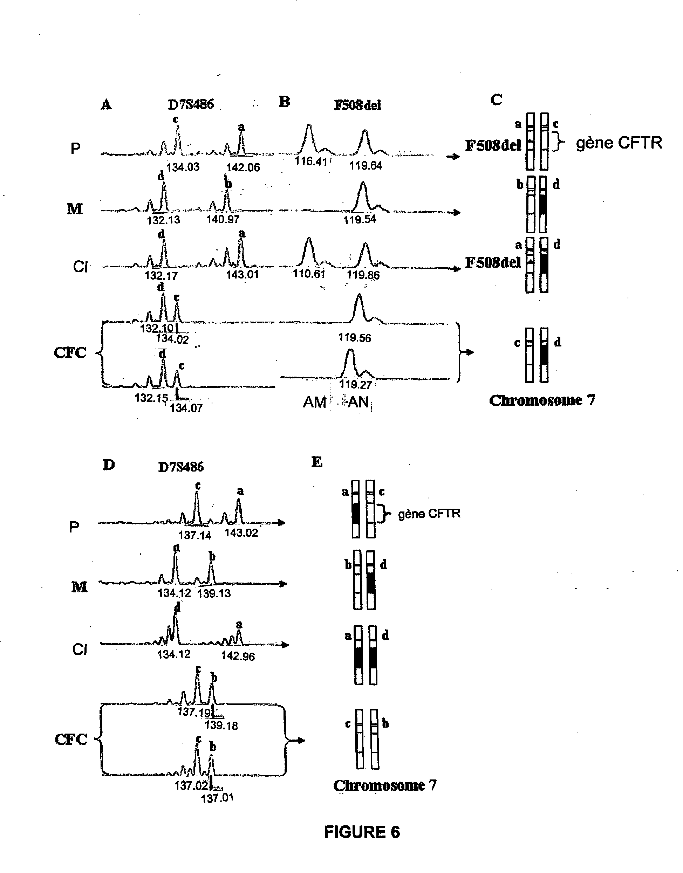

**Figure 6** **: Diagnostic prénatal non invasif de la mucoviscidose (CF) chez des couples présentant des mutations CFTR inconnues**
   **A, B, C** : Génotypage d'ADN du couple 11 et de leur enfant affecté avec le marqueur informatif D7S486 sur le chromosome 7, montrant que le père (P) et la mère (M) sont hétérozygotes. Le père porte les allèles c et a, tandis que la mère porte les allèles d et b. Leur enfant affecté (CI : cas index) porte les allèles d et a, qui doit donc être lié aux allèles CFTR mutés. Deux CFCs de la grossesse en cours portent les allèles d et c, démontrant que le foetus est porteur de la mucoviscidose, mais est non affecté.
   B : génotypage d'AND du locus F508 montrant que le père porte un allèle muté (AM) et un allèle normal (AN). Le cas index présente une hétérozygotie composée, et porte l'allèle Fdelta508 paternel et une mutation CFTR maternelle. Deux cellules foetales sont homozygotes pour l'allèle F508 normal. Prises ensemble, les données de A et B montrent que le foetus est porteur de la mutation CFTR maternelle. C : représentation schématique des allèles CFTR et des allèles STR liés à CFTR (a, b, c, d). bloc grisé = mutation CFTR inconnue.
   D, E : le génotypage STR d'ADN du couple 12 avec le marqueur D7S486 sur le chromosome 7 montre que le père et la mère sont hétérozygotes et que le cas index porte les allèles a et d D7S486, qui doivent donc être liés aux allèles CFTR mutés. Deux CFCs de la grossesse en cours portent les allèles c et d D7S486 liés aux allèles CFTR mutés, démontrant que le foetus est complètement normal.
   E. représentation schématique des allèles CFTR et des allèles STR liés à CFTR (a, b, c, d). Blocs grisés = allèles CFTR mutés.

### EXEMPLES

### Exemple 1: Diagnostic non invasif de la prédisposition à la mucoviscidose par l'enrichissement efficace de cellules trophoblastiques et l'analyse de mutations limitée aux génomes de cellules uniques révélées foetales par génotypage.

### 1-1 Méthode

Le sang périphérique de 12 femmes, à 11 à 13 semaines de grossesse, nécessitant un diagnostic prénatal de la mucoviscidose a été étudié par ISET (isolement selon la taille des cellules tumorales épithéliales/trophoblastiques).

Six ml de sang maternel (avant PVC) et 2 ml de sang paternel ont été collectés sur un tampon acide éthylènediaminetétraacétique. L'ADN paternel et l'ADN maternel ont été extraits à partir de 1 ml de sang et 1,5 ng ont été utilisés pour un allélotypage avec des amorces spécifiques des marqueurs microsatellites (STR) D7S480, D7S486, D16539, D16S3018, D21S1435 et D21S1437. Un ensemble d'amorces externes (out) et un d'amorces internes (in) (fluorescéinées) a été utilisé pour chaque marqueur STR (Cf. tableau 1 pour la séquence des amorces et les profils de PCR). Quatre ml de sang maternel ont été traités par ISET jusqu'à 3 heures après collecte, comme décrit antérieurement (Vona et al., 2000, 2002). Brièvement, les prélèvements sanguins ont été dilués à 1:10 avec le tampon ISET et traités par l'appareil d'ISET (Metagenex, Paris, France). Les grandes cellules de chaque ml de sang ont été concentrées sur une tache circulaire de 0,6 cm de diamètre sur la membrane d'ISET. Deux ml de chaque prélèvement de sang maternel traité par ISET ont été analysés. Après analyse immunohistochimique avec l'anticorps KL1 (Vona et al., 2002) pour identifier les cellules épithéliales et coloration à l'hématoxyline, une microdissection au laser d'une unique cellule a été effectuée à l'aide d'un Leica (AS LMD) et d'un microscope équipé d'un laser Nikon (TE 2000 U, Nikon France SA). Pour s'assurer qu'une seule cellule avait été collectée, nous avons pris des photographies de la cellule avant et après microdissection, et de la cellule microdisséquée sur le culot.

Chaque cellule collectée a été lysée dans 15 µl de tampon de lyse (Tris-HCl 100 mmol/l, pH 8, protéinase K 400 µg/ml) pendant 16 heures à 37°C, suivi de l'inactivation de la protéinase K à 94°C pendant 15 minutes. Après préamplification par extension des amorces (PEP) effectuée comme décrit antérieurement (Vona et al., 2000, 2002) dans un volume de 60 µl, nous avons utilisé des aliquotes de 6 µl pour des amplifications supplémentaires. Un génotypage des cellules uniques a été réalisé à l'aide d'amorces spécifiques des STR présentés comme informatifs par le génotypage de l'ADN maternel et paternel. Une autre aliquote de 6 µl de produit de PEP, obtenue à partir de cellules uniques, révélées foetales par génotypage par STR, a été utilisée pour rechercher l'allèle deltaF508 à l'aide d'amorces comportant le locus deltaF508. Une amplification a été effectuée dans 40 µl contenant 6 µl de produit de PEP, 10 mmol/l de Tris-HCl, 50 mmol/l de KCI, 25 mmol/l de MgCl₂, 200 µmol/l de chaque désoxynucléotide, 0,5 µM de chaque amorce externe et 2 U de Taq Gold (Applied Biosystems, Foster City, CA). 2 µl du premier produit de PCR ont de nouveau été amplifiés dans 40 µl à l'aide des amorces internes et du même protocole (Cf. tableau 1 pour la séquence des amorces et les profils de PCR). 1 µl du produit de PCR dilué à 1:20 a ensuite été mélangé à 13,5 µl de Hi-Di Formamide déionisé, 0,5 µl de marqueur Genescan 400 HD (ROX) (Applied Biosystems, Foster City, CA), et chargé sur un séquenceur automatique ABI Prism 3100 (Applied Biosystems, Foster City, CA). Les profils ont été analysés à l'aide des programmes Genescan et Genotypersoftware (Perkin Elmer, Foster City, CA).

**Tableau 1 : Amorces et Profils de PCR**

| **Nom de l'amorce** | **Numéro de la séquence** | **Séquence de l'amorce** | **Profils de PCR** |
|---|---|---|---|
| D7S480 out* | SEQ ID NO: 1 | F: 5'- AAAAACCCTGGCTTATGC-3' | 5 min 94°C,40 x (30 s 94°C, 30 s 58 °C, |
| | SEQ ID NO: 2 | R : 5'-AGCTACCATAGGGCTGGAGG-3' | 30 s 72 °C),5 min 72°C. |
| | | | |
| D7S480 in* | SEQ ID NO: 3 | F : 5'-CTTGGGGACTGAACCATCTT-3' | 5 min 94°C,40 x (30 s 94°C, 45 s 55 °C, |
| | SEQ ID NO: 4 | R : 5'-AGCTACCATAGGGCTGGAGG-3' | 30 s 72 °C),5 min 72°C. |
| | | | |
| D7S486 out* | SEQ ID NO: 5 | F: 5'- GGAATCTGTTCTGGCAATGGAT-3' | 5 min 94°C,40 x (30 s 94°C, 45 s 55 °C, |
| | SEQ ID NO: 6 | R: 5'- TTGCAATGAGCCGAGATCCTG-3' | 30 s 72 °C),5 min 72°C. |
| | | | |
| D7S486 in* | SEQ ID NO: 7 | F: 5'-AAAGGCCAATGGTATATCCC-3' | 5 min 94°C,40 x (30 s 94°C, 30 s 55 °C, |
| | SEQ ID NO: 8 | R: 5'GCCCAGGTGATTGATAGTGC-3' | 30 s 72 °C),5 min 72°C. |
| | | | |
| D16S539 out | SEQ ID NO: 9 | F: 5'- CAGATGCTCGTTGTGCACAA-3' | 5 min 94°C,40 x (30 s 94°C, 45 s 60 °C, |
| | SEQ ID NO: 10 | R: 5'- ATACCATTTACGTTTGTGTGTG-3' | 30 s 72 °C),5 min 72°C. |
| | | | |
| D16S539 in | SEQ ID NO: 11 | F: 5'-GATCCCAAGCTCTTCCTCTT-3' | 5 min 94°C,40 x (30 s 94°C, 30 s 58 °C, |
| | SEQ ID NO: 12 | R: 5'- ACGTTTGTGTGTGCATCTGT-3' | 30 s 72 °C),5 min 72°C. |
| | | | |
| D16S3018 out | SEQ ID NO: 13 | F: 5'- TGACAGTGCAGCTCATGGTC-3' | 5 min 94°C,40 x (30 s 94°C, 30 s 61 °C, |
| | SEQ ID NO: 14 | R: 5'- GGTCATTGGTCAAGGGCTGCT -3' | 30 s 72 °C),5 min 72°C. |
| | | | |
| D16S3018 in | SEQ ID NO: 15 | F: 5'-GGATAAACATAGAGCGACAGTTC-3' | 5 min 94°C,40 x (30 s 94°C, 30 s 58 °C, |
| | SEQ ID NO: 16 | R: 5'- AGACAGAGTCCCAGGCATT-3' | 30 s 72 °C),5 min 72°C. |
| | | | |
| D21S1435 out | SEQ ID NO: 17 | F: 5'-TTGACATTCTTCTGTAAGGAAGA-3' | 5 min 94°C,40 x (30 s 94°C, 30 s 58 °C, |
| | SEQ ID NO: 18 | R: 5'- AGGCTTGCCAAAGATATTAAAAG-3' | 45 s 72 °C),5 min 72°C. |
| D2151435 in | SEQ ID NO: 19 | F: 5'-CCCTCTCAATTGTTTGTCTACC-3' | 5 min 94°C,40 x (30 s 94°C, 30 s 58 °C, |
| | SEQ ID NO: 20 | R: 5' GCAAGAGATTTCAGTGCCAT-3' | 30 s 72 °C),5 min 72°C. |
| | | | |
| D21S1437 out | SEQ ID NO: 21 | F: 5'-TTGTGAATAGTGCTGCAATG-3' | 5 min 94°C,40 x (30 s 94°C, 45 s 60 °C, |
| | SEQ ID NO: 22 | R: 5' -ATGTACACTGACTTGTTTGAG-3' | 30 s 72 °C),5 min 72°C. |
| | | | |
| D21S1437 in | SEQ ID NO: 23 | F: 5'-ATGTACATGTGTCTGGGAAGG-3' | 5 min 94°C,40 x (30 s 94°C, 45 s 58 °C, |
| | SEQ ID NO: 24 | R: 5' -TTCTCTACATATTTACTGCCAACA-3' | 30 s 72 °C),5 min 72°C. |
| | | | |
| Delta F508 out | SEQ ID NO: 25 | F: 5'-TGGAGCCTTCAGAGGGTAAA-3' | 5 min 94°C,40 x (30 s 94°C, 30 s 55°C, |
| | SEQ ID NO: 26 | R: 5'- TGCATAATCAAAAAG TTT TCACA-3' | 30 s 72 °C),5 min 72°C. |
| | | | |
| Delta F508 in | SEQ ID NO: 27 | F: 5'-TCT GTT CTCAGT TTT CCTGG-3' | 5 min 94°C,40 x (30 s 94°C, 30 s 57°C, |
| | SEQ ID NO: 28 | R: 5'- TCT TAC CTC TTC TAG TTG GC-3' | 30 s 72 °C),5 min 72°C. |
| | | | |
| D7S490 out | SEQ ID NO: 29 | F: 5'- AAGTAATTCTCCTGCCTCAG-3' | 5 min 94°C,40 x (30 s 94°C, 30 s 58 °C, |
| | SEQ ID NO: 30 | R: 5'- AGCTACTTGCAGTGTAACAGCATTT -3' | 30 s 72 °C),5 min 72°C. |
| | | | |
| D7S490 in | SEQ ID NO: 31 | F: 5'- CCTTGGGCCAATAAGGTAAG-3' | 5 min 94°C,40 x (30 s 94°C, 30 s 55 °C, |
| | SEQ ID NO: 32 | R: 5'- AGCTACTTGCAGTGTAACAGCATTT-3' | 30 s 72 °C),5 min 72°C. |
| | | | |
| D7S523 out | SEO ID NO: 33 | F: 5'- GAATTATAACCGTAACTGATTC-3' | 5 min 94°C,40 x (30 s 94°C, 30 s 58 °C, |
| | SEQ ID NO: 34 | R:5'- GAGATAATGCTTGTCTGACTTC-3' | 30 s 72 °C),5 min 72°C. |
| | | | |
| D7S523 in | SEQ ID NO: 35 | F: 5'- CTGATTCATAGCAGCACTTG -3' | 5 min 94°C,40 x (30 s 94°C, 30 s 58 °C, |
| | SEQ ID NO: 36 | R:5'- AAAACATTTCCATTACCACTG-3' | 30 s 72 °C),5 min 72°C. |

| | | | |
|---|---|---|---|
| * Amorces utilisées pour le diagnostic indirect | | | |

### 2-2 Résultats

Chez les couples où aucun ou un seul des parents était porteur de l'allèle deltaF508, nous avons demandé des informations au Laboratoire de Génétique Médicale concernant un premier enfant affecté (cas index) et les amorces de STR informatifs situés sur le chromosome 7. Nous avons ensuite utilisé ces amorces pour amplifier l'ADN des cellules foetales et effectuer un diagnostic par la méthode indirecte (Cf. figures 2 et 3). Les analyses de séquences ont été effectuées à l'aide d'un kit de séquençage Big Dye-Terminator (Applied Biosystems, Foster City, CA) (Vona et al., 2002) après purification du produit de PCR sur colonnes Microspin S-400RH (Amersham Bioscience, Buckinghamshire, GB). Les prélèvements sanguins des mères des couples n°4 à n°12 (tableau 2) ont été analysés selon une approche totalement en aveugle par des chercheurs ignorant les résultats des PVC.

Au moins deux marqueurs informatifs (tableau 2), identifiés parmi ceux testés sur l'ADN paternel et maternel isolé à partir de cellules PBL (tableau 1), ont été utilisés pour l'allélotypage des cellules microdisséquées. Ceci nous a permis d'identifier au moins deux cellules foetales à partir de seulement 2 ml de sang maternel.

L'ADN des cellules foetales uniques a ensuite été analysé avec des amorces emboîtées comportant le locus deltaF508. Cette analyse peut révéler l'allèle deltaF508, qui est caractérisé par une délétion de 3 bp (CTT) (produit de PCR de 117 bp au lieu de 120 bp, Cf. figure 1), permettant de savoir si le foetus est affecté par la mucoviscidose (présence homozygote de l'allèle deltaF508), porteur de l'allèle deltaF508 (présence hétérozygote de l'allèle deltaF508), ou normal (absence homozygote de l'allèle deltaF508). Comme l'indique le tableau 2, la présence hétérozygote de l'allèle deltaF508 a été découverte dans toutes les cellules foetales isolées à partir du sang maternel de 7 femmes appartenant aux couples de porteurs de l'allèle deltaF508. Dans 6 cellules foetales, ce résultat a été confirmé par séquençage du produit de PCR spécifique de deltaF508 (figure 1). Les cellules foetales isolées à partir du sang maternel des 3 autres couples de porteurs de l'allèle deltaF508 se sont révélées être homozygotes pour l'absence de l'allèle deltaF508. Les cellules foetales isolées à partir du couple n°11 (figure 2) ont révélé l'absence homozygote de l'allèle deltaF508. La présence d'une mutation inconnue dans l'allèle paternel a été recherchée par amorces de STR informatifs sur le chromosome 7 (diagnostic indirect). L'analyse a prouvé la présence de l'allèle muté dans les cellules foetales analysées. Les cellules foetales isolées à partir du sang maternel du couple n°12 ont été testées avec des amorces de STR informatifs sur le chromosome 7 (Cf. tableau 2) afin de réaliser un diagnostic indirect. Les résultats ont montré que le foetus avait hérité des allèles non mutés à la fois maternel et paternel (figure 3 et tableau 2). Tous les résultats que nous avons obtenus sur des cellules foetales étaient cohérents avec les résultats obtenus par PVC.

**Tableau 2 : Diagnostic prénatal de la mucoviscidose sur des cellules foetales circulantes (méthode non invasive)**

| **Couple** | **Marqueurs informatifs** | **Nombre de cellules foetales testées** | **Diagnostic prénatal** | |
|---|---|---|---|---|
| | | | **Invasif (PVC)** | **Non invasif (ISET)** |
| | | | | |
| 1 | D7S486/ D16S539 | 3 | Hétérozygote Δ F508 | Hétérozygote Δ F508 |
| 2 | D16S539/D21S1435 | 2 | Homozygote NL | Homozygote NL |
| 3 | D7S486/ D16S3018 | 2 | Hétérozygote Δ F508 | Hétérozygote Δ F508 |
| 4 | D16S3018/ D21S1435 | 1 | Homozygote NL | Homozygote NL |
| 5 | D16S3018/D21S1437 | 2 | Hétérozygote Δ F508 | Hétérozygote Δ F508 |
| 6 | D16S539 / D16S3018 | 2 | Homozygote NL | Homozygote NL |
| 7 | D16S539/D21S1437 | 1 | Hétérozygote Δ F508 | Hétérozygote Δ F508 |
| 8 | D16S3018/ D21S1435 | 1 | Hétérozygote Δ F508 | Hétérozygote Δ F508 |
| 9 | D16S539/D16S3018 | 3 | Hétérozygote Δ F508 | Hétérozygote Δ F508 |
| 10 | D7S486/ D16S3018 | 2 | Hétérozygote Δ F508 | Hétérozygote Δ F508 |
| 11 | D7S486/ D16S3018/ D21S1435 | 2 | Hétérozygote MU² | Hétérozygote MU² |
| 12 | D7S480/ D7S486/ D16S3018 | 2 | Homozygote NL² | Homozygote NL² |
| | | | | |

| | | | | |
|---|---|---|---|---|
| ^{2:} Diagnostic indirect ; PVC : prélèvement de villosités choriales ; NL : normal ; MU : mutation inconnue | | | | |

### 3-3 Discussion

Nous prouvons ici la faisabilité d'un diagnostic prénatal non invasif de la mucoviscidose par l'enrichissement efficace de cellules trophoblastiques et l'analyse de mutations limitée aux génomes de cellules uniques révélées foetales par génotypage. Les résultats montrent que cette approche est valide à la fois pour des couples de porteurs de l'allèle deltaF508 et de porteurs de mutations inconnues à condition, dans ce dernier cas, que des informations sur un premier enfant affecté (cas index) soient disponibles. D'un point de vue technique, l'application d'analyses de cellules uniques à un diagnostic prénatal est limitée par le risque de perte d'allèle (ADO, *allele drop out*)*,* l'incapacité de la PCR à amplifier l'une des deux séquences alléliques. Nos tests ont confirmé que l'ADO est strictement liée à la séquence des amorces et au profil de PCR. Une fois que ces paramètres ont été définis, l'ADO n'est plus une limitation à notre dosage. Lors de l'étape de génotypage des cellules uniques, même si une ADO se produit (nous l'avons en fait observée deux fois), la seule conséquence est la perte d'une cellule foetale, sans risque d'effectuer un diagnostic erroné. Nous avons en fait confirmé le génotype foetal de toute la cellule micro-disséquée par deux marqueurs STR. Pour l'amplification de la mutation de la mucoviscidose dans des cellules foetales uniques, nous avons obtenu des résultats cohérents dans les cellules foetales analysées. A ce stade, il est possible de mélanger les produits de PEP obtenus à partir de deux cellules foetales ou plus, une procédure qui est connue pour éliminer le risque d'ADO. Cependant, dans ce travail, les résultats ont été obtenus sur des cellules foetales uniques, sans avoir à mélanger l'ADN de plusieurs cellules foetales uniques. Les données décrites ici dévoilent pour la première fois une approche fiable pour effectuer un diagnostic prénatal de la mucoviscidose sur des cellules foetales circulantes, offrant de manière sûre des choix prénataux aux parents risquant d'avoir un enfant affecté par la mucoviscidose.

### Exemple 2 : Filtration d'un échantillon de sang maternel pur ou dilué, de façon à concentrer sur un filtre selon leur taille certaines cellules circulantes et en particulier des cellules d'origine foetale et analyse des cellules retenues sur le filtre afin d'obtenir une présomption de leur origine foetale ou maternelle.

Les échantillons de sang ont été dilués 10 fois dans un tampon de filtration contenant 0,175% de saponine, 0,2% de paraformaldéhyde, 0,0372% d'EDTA et 0,1% de BSA, puis filtrés à l'aide d'un filtre calibré de polycarbonate présentant des pores calibrés cylindriques de 8 µm de diamètre. Les cellules retenues sur le filtre ont été regroupées sur un spot circulaire de 0,6 cm de diamètre. Après coloration à l'éosine et à l'hématoxyline, les spots ont été analysés au microscope et une photographie de chaque cellule a été prise à fort et faible agrandissement. La taille des cellules a été déterminée à l'aide du logiciel Adobe Photoshop, en prenant comme référence la taille de 8 µm des pores. Les photos permettent de retrouver les cellules au microscope Pixcell II Arcturus (Mountain View, CA). La figure 2 illustre l'analyse microscopique obtenue par cette méthode.

On a réalisé la microdissection de chaque cellule par capture au laser sans aucun traitement préalable du filtre. Afin de s'assurer qu'une seule cellule est collectée à chaque fois, on a pris des photographies du filtre avant et après la microdissection et de la cellule microdisséquée déposée sur la capsule (CapSure™ HS). La cellule a ensuite été lysée dans 15 µl de tampon de lyse (100 mM Tris-HCl pH 8, ,400 µg/ml proteinase K) pendant 16 heures à 37°C. Le lysat a été collecté après centrifugation et la protéinase K a été inactivée à 90°C pendant 10 minutes. Après une préamplification par extension d'amorces (PEP) comme décrit par Zhang *et al.* (voir plus haut), l'ADN a été précipité à l'éthanol et resuspendu dans 10 µl d'eau. Chaque échantillon a ensuite été testé, d'une part, avec les amorces décrites dans la présente demande.

### Exemple 3 : Etapes d'amplification.

Des PCR ont été mises en oeuvre sur un volume de mélange réactionnel de 20 µl contenant 2 µl de produit PEP (préamplification par extension d'amorces), 10 mM Tris-HCl, 50 mM KCI, 1,5 mM MgCl₂, 0,01% gélatine, 200 mM de chaque désoxynucléotide, 20 picomoles de chaque amorce utilisée et 1 U de Taq polymérase (Perkin-Elmer Cetus, Emeryville, CA). Après une étape de dénaturation initiale à 94°C pendant 5 minutes, on a procédé à 40 cycles d'amplification (94°C 30 s, 55 à 61 °C 30 à 45 s, 72°C 30 s) à l'aide des amorces externes selon l'invention et ce produit amplifié a de nouveau été amplifié à l'aide des amorces internes selon l'invention et du même protocole, puis on a réalisé une étape finale d'élongation à 72°C pendant 5 minutes dans un thermocycleur Perkin Elmer 9700 (cf tableau 1).

### Example 4 : la détection de mutations dans le gène CFTR héritées du père et de la mère dans des cellules foetales isolées du sang maternel permettent un diagnostic prénatal sûr de la mucoviscidose

Une approche multi-marqueurs STR a été développée, de sorte à accélérer l'étape de génotypage sur une seule cellule, et l'analyse de la mutation F508 a été menée sur les génomes de trois cellules trophoblastiques mis ensemble (*pooled genomes*), de sorte à éviter les problèmes de perte allélique (*allele drop out,* ADO).

### 1-1 Matériels et Méthode

### Prélèvement du sang et ISET

Six mL de sang maternel (avant PVC) et 1 mL de sang paternel ont été collectés sur un tampon acide éthylènediaminetétraacétique (EDTA). L'ADN paternel et l'ADN maternel ont été extraits à partir de 1 mL de sang et 1,5 ng ont été utilisés pour un allélotypage avec des amorces qui portent un marqueur fluorescéine, et qui sont spécifiques de marqueurs STR liés au locus CFTR (D7S480, D7S486, D7S490, D7S523) ou à d'autres loci génomiques (D16539, D16S3018, D21S1435 et D21S1437, cf. tableau 1 ci-dessus).

Les 5 mL restants de sang maternel ont été traités par ISET jusqu'à 3 heures après prélèvement, comme décrit précédemment, en utilisant l'appareil ISET (Metagenex, Paris, France ; www.metagenex.fr) et conservés à -20°C (Beroud et al., 2003 ; Vona et al. 2002). Trois mL de chaque échantillon de sang maternel traité par ISET (c'est-à-dire trois tâches - *spots -* sur le filtre) ont été analysés. Après analyse immunohistochimique avec l'anticorps KL1 (Vona et al., 2002) pour identifier des cellules épithéliales, une microdissection au laser sur cellule unique a été réalisée en utilisant le microscope équipé de laser Nikon TE 2000 U (Nikon, Paris, France et MMI, Zurich, Suisse). L'ISET enrichit en cellules épithéliales à partir du sang, mais aussi retient environ 0,02% de leucocytes du sang périphérique (Vona et al. 2002 ; Vona et al. 2000).

Ainsi, pour développer le protocole moléculaire pour le diagnostic prénatal non invasif de la mucoviscidose, le sang de 5 porteurs connus de Fdelta508 a été traité par ISET, et 75 cellules uniques de leucocytes ont été microdisséquées.

### Lyse cellulaire, pré-amplification par élongation des amorces et génotypage STR

Chaque cellule microdisséquée a été lysée dans 15 µl de tampon de lyse (Tris-HCl 100 mmol/L, pH 8, protéinase K 400 µg/ml) pendant 2 heures à 60°C, suivi de l'inactivation de la protéinase K à 94°C pendant 15 minutes. Après pré-amplification par extension des amorces (PEP) (Zhang et al., 1992), à la cellule lysée, ont été ajoutés 5µL d'une solution 400 µM d'amorces aléatoires (Kit genPEPₜₘ 75 OD, Genetix, Boston, USA), 6 µL de tampon PCR (25 mM MgCl2/gélatine (1 mg/mL), 100 mM tris-HCl, pH 8,3, 500 Mm KCL), 3 µL d'un mélange des 4 dNTPs (chacun à 2 mM) et 1 µL (5 U) of Taq polymérase (Applied Biosystem, Foster City, CA, USA) dans un volume final de 60 µL.

D'autres amplifications STR (cf. tableau 1 ci-dessus) ont été réalisées dans 40 µL contenant 4 µL de produit PEP, 10 mM de Tris-HCl, 50mM de KCI, 2,5 mM MgCl₂, 200 µM de chaque désoxynucleotide, 0,5 µM de chaque amorce et 2 U de Taq Gold (Applied Biosystems, Foster City, CA, USA). Un µL du produit PCR dilué au 1 :20 a alors été mélangé à 13,5 µL de formamide déionisé Hi-Di et à 0,5 µL de marqueur Genescan 400 HD (ROX) (Applied Biosystems), et a été placé dans un séquenceur automatique ABI Prism 3100 (Applied Biosystems). Les profils ont été analysés en utilisant les programmes Genescan et Genotyper (Applied Biosystems).

**Tableau 3 : Taux d'efficacité PCR et de précision d'amplification (PA) en utilisant des amorces STR- et F508-spécifiques**

| | **Nombre de tests** | **Amorces** | **Efficacité PCR** | **Valeur p** | **PA (%)** | **ADO (%)** | **Valeur p** |
|---|---|---|---|---|---|---|---|
| | | | | | | | |
| **PCR cellule individuelle** | **75** | **F508** | 67(90%) | | 61(91%) | 6(9%) | |
| **PCR sur groupes^{a}** | **30** | **F508** | 30 (100%) | <0,001^{b} | 30(100%) | 0 (0%) | <0,001^{c} |
| | | | | | | | |
| **PCR cellule individuelle** | **75** | **M1** | 58 (77%) | 0,1^{d} | 42 (72%) | 16 (28%) | 0,2^{d} |
| | | **M2** | 54 (72%) | 0,9^{e} | 38 (70%) | 16 (30%) | 0,9^{e} |
| | | **M3** | 52 (69%) | 0,6^{f} | 39 (75%) | 13 (25%) | 0,4^{f} |
| | | **M1+M2+M3^{g}** | 71 (95%) | <0,001^{def} | 67(94%) | 4 (6%) | <0,001 ^{def} |

| | | | | | | | |
|---|---|---|---|---|---|---|---|
| Abréviations: STR, *Short Tandem Repeat* ; PA, précision d'amplification; ADO (*allele drop out*) perte allélique; M1, D16S3018; M2, D16S539; M3, D21S1435. ^{a} mélange (*pooling*) de 3 produits PEP à partir de génomes de cellules individuelles amplifiables ; ^{b} vs. efficacité PCR F508 sur cellule individuelle ; ^{c} vs. précision PCR F508 sur cellule individuelle; ^{d} vs. M2 ; ^{e} vs. M3 ; ^{f} vs. M1 ; ^{g} marqueurs testés en amplifications indépendantes. | | | | | | | |

Nous avons testé l'efficacité du génotypage sur cellule individuelle en calculant le taux d'échec PCR et le taux de perte allélique (ADO) dans des tests STR réalisés sur 75 leucocytes micro-disséqués. Nous avons utilisé trois marqueurs hétérozygotes (cf. tableau 3 ci-dessus), donnant deux allèles distinguables. Le taux d'efficacité PCR a été calculé comme étant le taux de cellules qui donnent un produit PCR, et le taux de précision PCR a été calculé comme le taux de cellules qui présente les deux allèles avec au moins un des trois marqueurs STR utilisés.

### Protocole pour l'amplification du locus F508 sur cellule individuelle

Des fractions aliquotes de 4µL ont été prélevées des 60µL du produit PEP pour amplifications par PCR en utilisant des amorces fluorescéinées couvrant le locus F508 (cf. tableau 1 ci-dessus). Les génomes de cellules individuelles ont été testés de manière individuelle (4µL de produit PEP par amplification), et par groupes de trois, en mélangeant 30µL du produit PEP et en prenant 12µL (à partir des 90µL) pour l'amplification de F508. Les amplifications ont été réalisées dans 40µL contenant 4µL du produit PEP (ou 100µL contenant 12µL du produit PEP), 10mM de Tris-HCl, 50mM de KCl, 2,5mM de MgCl₂, 200µL de chaque désoxynucléotide, 0,5µL de chaque amorce et 2 U de Taq Gold (Applied Biosystems, Foster City, CA, USA). Ce protocole permet d'éviter les étapes de purification d'ADN qui présentent un haut risque de perdre des copies d'ADN. 1µL du produit PCR dilué à 1:20 a alors été mélangé avec 13,5µL de formamide déionisé Hi-Ti et 0,5µL de marqueurs (ROX) Genescan 400 HT (Applied biosystems) et placé dans un séquenceur automatique ABI Prism 3100 (Applied Biosystems). Les profils ont été analysés en utilisant les programmes Genescan et Genotyper (Applied Biosystems).

### Application du protocole pour le diagnostique prénatal non invasif de la mucoviscidose à 12 femmes enceintes

Nous avons testé le sang périphérique de 12 femmes qui présentaient une grossesse de 11 à 13 semaines, et qui avaient demandé un diagnostic prénatal de la mucoviscidose, y compris deux femmes qui avaient eu un précédent enfant présentant une hétérozygotie composée pour la mucoviscidose (1 cas dit d'index ; couples 11 et 12, tableau 4). Toutes les femmes ont donné leur consentement éclairé à cette étude, qui a été approuvé par le comité local d'éthique.

**Tableau 4 : diagnostic pré-natal de la mucoviscidose (fibrose cystique, CF) par analyse génétique de cellules foetales circulantes et par prélèvement de villosités choriales**

| **Couple** | **Marqueurs STR informatifs** | **Nbre de CFCs testées** | **Génotype CF sur CFC individuelles^{b}** | **Génotype CF sur groupes de 3 CFCs^{c}** | **Génotype CF par CVS** | **Statut foetal** |
|---|---|---|---|---|---|---|
| 1^{a} | D7S486/ D16S539/D21S1435 | 4 | F508del/N | F508del/N | F508del/N | porteur CF |
| 2^{a} | D16S539/D21S1435/ D21 S 1437 | 3 | N/N | N/N | N/N | sain |
| 3^{a} | D7S486/D16S3018/ D21S1437 | 3 | F508del/N | F508del/N | F508del/N | porteur CF |
| 4^{a} | D16S539/D16S3018/D21S1435 | 4 | N/N | N/N | N/N | sain |
| 5^{a} | D16S3018/D21S1437/D21S1435 | 3 | F508del/N | F508del/N | F508del/N | porteur CF |
| 6^{a} | D7S480/D16S539/D16S3018 | 3 | N/N | N/N | N/N | sain |
| 7^{a} | D16S539/D21S1437/D21S1435 | 4 | F508del/N | F508del/N | F508del/N | porteur CF |
| 8^{a} | D7S480/D16S3018/D21S1435 | 3 | F508del/F508del | F508del/F508del | F508del/F508del | affecté |
| 9^{a} | D16S539/D16S3018/ D21S1435 | 3 | F508del/N | F508del/N | F508del/N | porteur CF |
| 10^{a} | D7S486/ D21S1435/ D21S1437 | 3 | F508del/N | F508del/N | F508del/N | porteur CF |
| 11^{de} | D7S486/D7S490/D7S523 | 4 | Mut/N | | Mut/N | porteur CF |
| 12^{d} | D7S480/D7S486/D7S523 | 3 | N/N | | N/N | sain |

| | | | | | | |
|---|---|---|---|---|---|---|
| Abréviations: ISET (*Isolation by Size of Epithelial Tumor*/*Trophoblastic cells*) isolement selon la taille des cellules tumorales épithéliales / trophoblastiques ; CVS (*chorionic villus sampling*) prélèvement de villosités choriales ; F508del/N, avec allèle Fdelta508 hétérozygote ; N/N, avec allèles normaux homozygotes ; F508del/F508del, avec allèles Fdelta508 homozygotes ; Mut/N, avec allèle muté hétérozygote. ^{a} porteurs Fdelta508 ; ^{b} une amplification précise et des résultats cohérents ont été obtenus dans toutes les CFCs testées ; ^{c} mélange (*pooling*) des produits de pré-amplification par élongation d'amorces (PEP) de trois CFCs ; ^{d} porteurs de mutations inconnues ; ^{e} un parent est un porteur Fdelta508. | | | | | | |

Dans des cellules épithéliales circulantes qui se sont révélées être foetales par génotypage STR, on a recherché de manière individuelle, et par groupe de trois cellules, la présence de mutations DeltaF508. Les tests de groupes ont été réalisés, comme décrits ci-dessus, en mélangeant 30µL du produit PEP de trois cellules foetales par échantillon sanguin, et ensuite en prenant 12µL (à partir des 90µL pour l'amplification F508). Les analyses de séquences ont été réalisées en utilisant le kit de séquençages Big Dye Terminator (Applied Biosystems) (Vona et al. 2002) après purification du produit PCR sur des colonnes MicroSpin S-400RH (Amerscham Bioscience, Buckinghamshire, UK). Chez les couples dans lequel aucun, ou seulement un des parents, était porteur de la mutation deltaF508, l'analyse de cellules foetales a été réalisée par une méthode indirecte, en utilisant des marqueurs polymorphes STR informatifs liés au locus CFTR sur le chromosome 7, permettant ainsi de comparer le cas index et les haplotypes du foetus.

Les tests non invasifs ont été réalisés dans le département de Biochimie de l'Hôpital Necker - enfants malades en utilisant une approche complètement à l'aveugle par des opérateurs qui n'étaient pas informés des résultats de l'analyse invasive, qui a été réalisée dans le département de Génétique Médicale du même Hôpital.

### Analyses statistiques

Le test du Chi2 a été utilisé pour les analyses statistiques de fréquences entre groupes. Une valeur p de moins de 0,05 a été considérée comme significative.

### 2-2 Résultats

### Génotypage STR sur cellule unique

Pour optimiser le protocole de génotypage STR, nous avons analysé les génomes de 75 leucocytes individuels, en utilisant un ou trois marqueurs STR hétérozygotes (M1, M2 et M3 (cf. tableau 3 ci-dessus)). Ceci nous a permis de distinguer les deux allèles, et de déterminer le taux de perte allélique (ADO) à l'étape de génotypages (cf. tableau 3). En utilisant seulement un marqueur, M1 ou M2 ou M3, nous avons obtenu une efficacité PCR (taux de cellules donnant un produit PCR) de 77%, 72%, 69% respectivement, et une précision PCR (taux de cellules présentant les deux allèles) de 72%, 70% et 75%, respectivement. Lorsque nous avons combiné les résultats obtenus avec les trois marqueurs STR, l'efficacité PCR (taux de cellules donnant un produit PCR avec au moins un des trois marqueurs STR utilisés) était de 95%, et la précision PCR (taux de cellules présentant les deux allèles avec au moins un des trois marqueurs STR utilisés) a augmenté significativement à 94% (cf. tableau 3). Ces données montrent que, à l'étape de génotypage de cellules micro-disséquées individuelles, l'efficacité et la précision PCR sont significativement augmentées en réalisant un génotypage STR informatif multi-marqueurs.

### Amplification du locus F508 du gène CFTR

Pour développer un protocole qui surmonte le problème de la perte allélique (ADO), nous avons utilisé l'ADN de 75 leucocytes individuels provenant de porteurs deltaF508 prouvés. Dans ce cas, l'allèle normal et l'allèle muté sont distinguables car ils ont une taille différente (120pb et 117pb, respectivement) (voir par exemple figure 5A') dans les amplifications sur cellule individuelle, nous avons détecté au moins un allèle F508 dans 67 des 75 cellules. Dans les 8 autres cellules, il n'y avait pas de signal, ce qui indique que l'étape de pré-amplification était insuffisante. Ainsi, l'efficacité PCR était de 90%. Parmi les 67 cellules positives à la PCR, nous avons observé une perte allélique (ADO) dans six de ces cellules (9%), et une amplification précise des deux allèles dans les 61 cellules restantes, ce qui donne une précision PCR de 91% (cf. tableau 3). Comme la perte allélique (ADO) est un évènement stochastique, et affecte l'un ou l'autre allèle à une fréquence d'environ 1 sur 10 analyses (0,1), elle affectera le même allèle à une fréquence de 1/20 (0,05). Nous en avons donc déduit que si nous mélangions l'ADN de 3 cellules foetales ou plus, la fréquence de perte allélique affectant le même allèle tomberait à 0,0001 (0,05 x 0,05 x 0,05 = 0,000125). Nous avons donc mélangé en aveugle (par un opérateur ne connaissant pas les résultats précédents) les produits PEP de 67 échantillons d'ADN de leucocytes dans 30 bains aléatoires de trois, et avons réalisé l'amplification F508 et l'analyse de fragment comme décrit ci-dessus. Dans ce cas, nous avons observé de manière cohérente l'absence de perte allélique (cf. tableau 3 ci-dessus) dans l'ensemble des 30 tests.

Lorsque nous avons comparé les résultats des cellules individuelles avec ceux des échantillons mélangés, nous avons observé que, dans 3 cas, nous avions mélangé l'ADN de deux cellules qui, lorsqu'elles étaient analysées individuellement donnaient une perte allélique, et l'ADN d'une cellule, qui lorsqu'elle était analysée de manière individuelle, présentait les deux allèles. Le résultat du mélange des trois échantillons correspond à une absence de perte allélique. Dans un cas, nous avons mélangé trois cellules qui individuellement conduisaient à une perte allélique dans le test sur cellule individuelle, mais qui lorsqu'elles sont mélangées ensemble, ne conduisaient pas à une perte allélique. Nous avons alors mélangé l'ADN de six cellules présentant une perte allélique dans neuf bains de trois. Les résultats montrent une absence de perte allélique dans tous les tests réalisés. Nous expliquons cette observation par le fait que, dans les analyses en groupes, les probabilités d'inclure des séquences d'ADN provenant d'un seul (et du même) des deux allèles dans le mélange complet est, comme calculé précédemment, très faible (1/10 000). Nous en avons conclu que, en mélangeant 30 µl de produits PEP de trois cellules provenant d'un foetus individuel et en prenant 12 µl pour la PCR, nous pouvons augmenter de manière très marquée la probabilité d'avoir des séquences bi-alléliques et d'avoir un test fiable pour détecter la présence d'un allèle deltaF508 dans le diagnostic prénatal non invasif de la mucoviscidose.

### Protocole pour le diagnostic prénatal non invasif de la mucoviscidose (Figure 4).

En se basant sur les résultats obtenus, nous avons défini le protocole suivant pour le diagnostic prénatal non invasif de la mucoviscidose. Les ADN extraits du sang maternel (1 mL) et du sang paternel (1 mL) sont comparés en utilisant des amorces STR pour identifier des marqueurs STR informatifs. L'ISET est utilisé pour enrichir en trophoblastes foeto-circulants à partir du sang maternel (5 mL). Les cellules trophoblastiques, identifiées par immunomarquage au KL1 et par morphologie cellulaire, sont ensuite récoltées de manière individuelle à partir du filtre ISET par microdissection à capture laser (LCM, *laser capture microdissection),* et leur ADN est ensuite analysé après lyse cellulaire. Le génome complet des cellules individuelles est tout d'abord amplifié par pré-amplification par élongation d'amorces aléatoires (PEP) (volume final : 60 µL). Des fractions aliquotes du produit PEP (4µL) ont été utilisées pour le génotypage avec 3 marqueurs STR informatifs pour déterminer si l'ADN est d'origine foetale (ayant à la fois les allèles maternels et paternels) ou d'origine maternelle. Les marqueurs STR informatifs sur le chromosome 7 permettent un diagnostic indirect de la mucoviscidose (si un cas d'index est disponible). Des fractions aliquotes (30 µL) du produit PEP de trois cellules foetales sont mélangées, et 12 µL sont utilisés pour l'amplification avec des amorces spécifiques de F508 ce qui permet d'éviter le risque de perte allélique (ADO) et de réaliser de manière fiable le diagnostic de la mucoviscidose.

### Application du protocole à 12 couples présentant un risque d'avoir un enfant avec la mucoviscidose.

Nous avons ensuite appliqué cette méthode améliorée aux cellules sanguines prélevées de mères qui présentaient un risque d'avoir un foetus affecté par la mucoviscidose. Les analyses de génotypage nous ont permis d'identifier au moins trois cellules foetales à partir de 3 mL de chaque sang maternel (voir par exemple en Figure 5, les éléments A, B et C). Ces cellules foetales ont été testées de manière individuelle, et également en groupes. Dans les groupes 1 à 10 (cf. Tableau 4 ci-dessus, cf. Figure 5) où les deux parents sont porteurs de deltaF508, nous avons étudié l'ADN extrait de cellules foetales avec des amorces couvrant le locus F508 (voir Figure 5). Cet essai a permis d'identifier la mutation deltaF508 qui est caractérisée par une délétion de trois paires de bases (CTT) qui donne un produit PCR de 117 pb au lieu de 120 pb (voir figures 5 A', B' et C'). Nous avons ainsi identifié des foetus hétérozygotes portant la mutation deltaF508 (couples 1, 3, 5, 7, 9 et 10) (Voir Figure 5, élément A'), des foetus homozygotes (couples 2, 4 et 6) (Figure 5, élément B'), et un foetus mutant homozygote pour la mucoviscidose (couple 8) Figure 5 élément C'). Dans six cellules foetales individuelles, les résultats que nous avons obtenus en génotypant par génotypage ont été confirmés en séquençant le produit de PCR spécifique du locus F508 (cf. Figure 5, éléments A", B" et C"). L'analyse de séquences n'a pas été réalisée de manière systématique, étant donné que les données obtenues par génotypages étaient extrêmement claires. Nous avons obtenu les mêmes résultats en testant de manière individuelle chaque CFC (cf. Tableau 4 ci-dessus) et en mélangeant les trois CFCs identifiéss dans chaque échantillon maternel. La fréquence de perte allélique était donc plus faible dans les échantillons provenant de CFCs (0/33) que ce que nous avions observé sur les leucocytes (6/67). Nous n'avons pas observé de perte allélique dans les groupes de produits PEP à partir des groupes de produit PEP de trois cellules foetales.

Nous avons aussi montré que nous pouvons réaliser le diagnostic prénatal non invasif de la mucoviscidose dans des couples présentant des mutations CFTR inconnues, dans la mesure où l'ADN d'une fratrie affectée par la mucoviscidose (cas d'index) est disponible pour identifier les allèles STR liés aux allèles CFTR mutés. Chez le couple 11 (cf. Tableau 4), le père était un porteur de la mutation deltaF508, et la mère était porteuse d'une mutation inconnue (cf. Figure 6 éléments A, B et C). Des analyses de CFCs individuelles avec des amorces STR informatives liées au locus CFTR sur le chromosome 7 (diagnostic indirect) (cf. Figure 6 A), et par des amorces spécifiques du locus F508 (voir Figure 6 B), montrent l'absence de la mutation deltaF508, et la présence sous forme hétérozygote de l'allèle maternel portant la mutation inconnue qui a été identifiée par sa présence dans le génome du cas d'index. Ainsi, le foetus était un porteur de la mutation maternelle inconnue. Chez le couple 12, le père et la mère étaient porteurs de la mutation inconnue (cf. Figure 6 D et E). Les analyses de cellules foetales avec des amorces STR informatiques sur le chromosome 7 (diagnostic indirect) ont montré l'absence homozygote des allèles mutées, précédemment identifiés dans le génome du cas d'index. Ainsi, le foetus était complètement normal.

Ce travail a entièrement été réalisé selon un protocole en aveugle, et des résultats cohérents ont été obtenus par la méthode non invasive ISET-CF et par la méthode invasive réalisée sur des échantillons de villosités choriales (CVS) par une équipe indépendante (voir Tableau 4 ci-dessus).

### 3-3 Discussion

Notre étude montre qu'un diagnostic prénatal non invasif de la fibrose cystique est possible, et peut être appliqué en routine dans le cadre de dispositifs cliniques. En réalité, l'application clinique de notre méthode aux cellules foetales isolées à partir du sang de 12 mères ayant un quart de risque de donner naissance à un enfant affecté par la fibrose cystique, s'est, dans un protocole en aveugle, avérée être une méthode de diagnostic fiable dans tous les cas, avec identification précise des mères dont les foetus sont sains, ou porteurs, ou affectés.

La méthode implique l'isolement de cellules foetales circulantes (CFCs) par ISET et microdissection laser, le génotypage pour déterminer la présence de marqueurs paternels (c'est-à-dire pour confirmer l'origine foetale de ces cellules), et l'analyse de la mutation d'un pool de trois cellules dont le caractère foetal avait été prouvé génétiquement. Les cellules foetales peuvent être isolées de toutes les mères sans risque supplémentaire de fausse couche, et l'analyse de la mutation peut être réalisée sur des pools de cellules dont on a prouvé le caractère foetal, ramenant presque à zéro, et de façon significative, la probabilité d'une ADO (perte allélique). Ces caractéristiques rendent le test fiable, et potentiellement applicable en clinique comme une alternative sûre aux procédures de diagnostic prénatal invasives.

Des approches antérieures en vue du développement de protocoles de routine pour le diagnostic prénatal non invasif avaient échoué du fait de la faible efficacité des méthodes utilisées pour enrichir et/ou identifier des cellules foetales circulantes (CFCs) (Bianchi, 1999 ; Bianchi, et al., 2002). De plus, les tests reposant sur le foetus portant un chromosome Y pour identifier des cellules foetales comme étant différentes du fonds cellulaire maternel ne sont pas applicables aux foetus féminins.

L'ADN foetal dépourvu de cellules dans le plasma maternel, correspond à 3-5 % de l'ADN plasmatique total (Lo, et al., 1998), et permet la détermination du sexe foetal, du statut foetal de rhésus D et des mutations ponctuelles foetales héritées du père dès lors que cet ADN n'existe pas dans le patrimoine génétique maternel (Li, et al., 2005 ; Li, et al., 2004). Cette approche, cependant, ne peut pas s'appliquer en routine pour le diagnostic prénatal de désordres récessifs tels que la fibrose cystique qui, par définition, requiert l'étude des mutations héritées de la mère et du père.

Notre nouvelle approche surmonte tous les obstacles du diagnostic prénatal non invasif de la fibrose cystique. Les rares trophoblastes circulants, qui sont considérés comme ne persistant pas après l'accouchement (Bianchi, et al., 1996) sont enrichis par ISET, parce qu'ils sont plus grands que les leucocytes du sang périphérique. Cet enrichissement est tellement efficace que les cellules foetales, 3 ou plus, ont été trouvées dans un échantillon unique de seulement 2-4 mL de sang pris chez toutes les mères du groupe de 51 testées jusqu'à présent dans notre laboratoire. Le génotypage de cellule individuelle après microdissection au laser permet d'identifier des cellules foetales de façon univoque au moyen de la contribution bi-parentale à leur génome. Au stade du génotypage de cellule individuelle, même si une ADO survient, la conséquence est que l'ADN de la cellule individuelle est incomplètement caractérisé, et en conséquence écarté. Il n'y a pas de risque de réaliser un diagnostic incorrect. Cependant, nous montrons ici que le génotypage de cellules individuelles en utilisant 3 marqueurs STR, réduit fortement le taux d'ADO, en accroissant l'efficacité du génotypage de cellule individuelle et en accélérant le procédé de diagnostic. Finalement, le fait de réunir (pooler) la moitié du produit de pré-amplification par élongation d'amorces (PEP) (30 µL de 60 µL) de 3 cellules foetales et le fait de prendre 12 µL pour l'amplification F508, permet de réaliser une analyse de mutation sur l'ADN de cellule foetale « pure », minimisant le diagnostic faux positif dû à la perte d'un allèle normal par ADO. Il a été préalablement démontré que le mélange d'ADN de 2 ou plus cellules uniques prévient presque complètement le risque d'ADO (Piyamongkol, et al., 2003), mais ces études ont été réalisées sur l'ADN cellulaire total (de cellules fraîches) qui n'avait pas été amplifié par PEP. Dans notre protocole, nous utilisons des aliquotes de produits PEP, rendant possible la réalisation de plusieurs analyses de PCR sur un génome de cellule individuelles, et sur un pool de génomes de plusieurs cellules à partir d'un foetus unique.

Dans notre expérience, le protocole optimisé de PEP inclut le degré de conservation d'ADN (nous conservons les filtres ISET pour le diagnostic prénatal non invasif à -20°C), le traitement avec la protéinase K pour lyser les protéines de cellules (plusieurs protocoles ont été testés de façon comparée), et la qualité des amorces dégénérées utilisées pour la PEP (tout les lots d'amorces sont vérifiés avant leur utilisation dans le protocole de diagnostic prénatal non invasif). Nous confirmons que la PEP n'empêche pas l'apparition d'ADO (Hahn, et al., 1998), mais nos résultats vont plus loin et montrent que l'ADO est empêchée par le mélange de produits de PEP mélangés sous forme de grande partie aliquote.

En réalité, les résultats obtenus en analysant 75 cellules testées individuellement et en pools de 3 cellules ont montré que la probabilité qu'une ADO, qui se produit de façon aléatoire, fasse échec à l'amplification du même allèle dans toutes les trois cellules poolées est extrêmement faible (1/10000). Dans la mesure où dans des protocoles de routine, le diagnostic prénatal non invasif pourrait être répété dans 5 à 10 pools de 3 cellules foetales à partir d'un échantillon de sang de 10 mL, et dans la mesure où le test pourrait être également répété dans 2 échantillons de sang supplémentaires pris chez la même mère, le risque de diagnostic erroné pourrait être proche de zéro.

Dans le test de cellule unique, nous avons observé une plus forte incidence d'ADO parmi les 67 lymphocytes (9 %) que parmi les 33 cellules foetales (0 %) analysées par PCR spécifique pour F508. Nous pensons que la nature compacte de l'ADN des lymphocytes pourrait expliquer leur plus fort taux d'ADO, si l'ADN de ces cellules est moins accessibles aux amorces de PCR.

Nous démontrons ici que le diagnostic prénatal non invasif de la fibrose cystique est possible même lorsque les deux parents portent des mutations CFTR inconnues (diagnostic indirect), dès lors que l'ADN d'un cas index (un enfant affecté du même couple) est disponible et que des amorces informatives STR sur le chromosome 7, où le gène CFTR est localisé, sont identifiées. En pratique, l'ADN d'un cas index est en général disponible parce que l'enfant affecté est très souvent l'indication unique que les parents sont porteurs des mutations de CFTR. Dans ce diagnostic indirect, il n'y a pas de nécessité de pooler les cellules foetales pour éviter les erreurs parce que les allèles STR à la fois maternels et paternels, requis pour le diagnostic doivent être visualisés. Ainsi, la présence des deux allèles assure l'absence d'ADO et le diagnostic fiable.

En conclusion, nous avons développé un protocole basé sur l'ISET offrant un diagnostic prénatal fiable et sûr du statut de patients sains, porteurs ou affectés par la fibrose cystique.

Cette stratégie optimisée qui peut être accélérée par une microdissection laser automatisée et le développement de kits pour l'analyse PCR spécifique, devrait permettre l'accès du diagnostic prénatal non invasif de la fibrose cystique en application clinique.

### 3-4 Références bibliographiques de l'exemple 4

Balinsky W, Zhu CW. 2004. Pediatric cystic fibrosis: evaluating costs and genetic testing. J Pediatr Health Care 18(1):30-4.
Beroud C, Karliova M, Bonnefont JP, Benachi A, Munnich A, Dumez Y, Lacour B, Paterlini-Brechot P. 2003. Prenatal diagnosis of spinal muscular atrophy by genetic analysis of circulating fetal cells. Lancet 361(9362):1013-4.
Bianchi DW. 1999. Fetal cells in the maternal circulation: feasibility for prenatal diagnosis. Br J Haematol 105(3):574-83.
Bianchi DW, Simpson JL, Jackson LG, Elias S, Holzgreve W, Evans MI, Dukes KA, Sullivan LM, Klinger KW, Bischoff FZ and others. 2002. Fetal gender and aneuploidy détection using fetal cells in maternal blood: analysis of NIFTY I data. National Institute of Child Health and Development Fetal Cell Isolation Study. Prenat Diagn 22(7):609-15.
Bianchi DW, Zickwolf GK, Weil GJ, Sylvester S, DeMaria MA. 1996. Male fetal progenitor cells persist in maternal blood for as long as 27 years postpartum. Proc Natl Acad Sci U S A 93(2):705-8.
Ciarleglio LJ, Bennett RL, Williamson J, Mandell JB, Marks JH. 2003. Genetic counseling throughout the life cycle. J Clin Invest 112(9):1280-6.
Fink L, Collins FS. 1997. The Human Genome Project: view from the National Institutes of Health. J Am Med Womens Assoc 52(1 ):4-7, 15.
Garvin AM, Holzgreve W, Hahn S. 1998. Highly accurate analysis of heterozygous loci bysingle cell PCR. Nucleic Acids Res 26(15):3468-72.
Hahn S, Garvin AM, Di Naro E, Holzgreve W. 1998. Allele drop-out can occur in alleles differing by a single nucleotide and is not alleviated by preamplification or minor template increments. Genet Test 2(4):351-5.
Hahn S, Zhong XY, Holzgreve W. 2002. Single cell PCR in laser capture microscopy. Methods Enzymol 356:295-301.
Hahn S, Zhong XY, Troeger C, Burgemeister R, Gloning K, Holzgreve W. 2000. Current applications of single-cell PCR. Cell Mol Life Sci 57(1):96-105.
Kerem B, Rommens JM, Buchanan JA, Markiewicz D, Cox TK, Chakravarti A, Buchwald M, Tsui LC. 1989. Identification of the cystic fibrosis gene: genetic analysis. Science 245(4922):1073-80.
Li Y, Di Naro E, Vitucci A, Zimmermann B, Holzgreve W, Hahn S. 2005. Detection of paternally inherited fetal point mutations for beta-thalassemia using size-fractionated cell-free DNA in maternal plasma. Jama 293(7):843-9.
Li Y, Holzgreve W, Page-Christiaens GC, Gille JJ, Hahn S. 2004. Improved prenatal détection of a fetal point mutation for achondroplasia by the use of size-fractionated circulatory DNA in maternal plasma--case report. Prenat Diagn 24(11):896-8.
Lo YM, Tein MS, Lau TK, Haines CJ, Leung TN, Poon PM, Wainscoat JS, Johnson PJ, Chang AM, Hjelm NM. 1998. Quantitative analysis of fetal DNA in maternal plasma and serum: implications for noninvasive prenatal diagnosis. Am J Hum Genet 62(4):768-75.
Nasis O, Thompson S, Hong T, Sherwood M, Radcliffe S, Jackson L, Otevrel T. 2004. Improvement in sensitivity of allele-specific PCR facilitates reliable noninvasive prenatal détection of cystic fibrosis. Clin Chem 50(4):694-701.
Piyamongkol W, Bermudez MG, Harper JC, Wells D. 2003. Detailed investigation of factors influencing amplification efficiency and allele drop-out in single cell PCR: implications for preimplantation genetic diagnosis. Mol Hum Reprod 9(7):411-20.
Schmidtke J. 1998. A Commentary on the NIH Consensus Development Statement 'Genetic Testing for Cystic Fibrosis'. Community Genet 1(1):53-6.
Vona G, Beroud C, Benachi A, Quenette A, Bonnefont JP, Romana S, Dumez Y, Lacour B, Paterlini-Brechot P. 2002. Enrichment, immunomorphological, and genetic characterization of fetal cells circulating in maternal blood. Am J Pathol 160(1):51-8.
Vona G, Sabile A, Louha M, Sitruk V, Romana S, Schutze K, Capron F, Franco D, Pazzagli M, Vekemans M and others. 2000. Isolation by size of epithelial tumor cells : a new method for the immunomorphological and molecular characterization of circulatingtumor cells. Am J Pathol 156(1):57-63.
Watson MS, Cutting GR, Desnick RJ, Driscoll DA, Klinger K, Mennuti M, Palomaki GE, Popovich BW, Pratt VM, Rohlfs EM and others. 2004. Cystic fibrosis population carrier screening: 2004 revision of American College of Medical Genetics mutation panel. Genet Med 6(5):387-91.
Zhang L, Cui X, Schmitt K, Hubert R, Navidi W, Arnheim N. 1992. Whole genome amplification from a single cell: implications for genetic analysis. Proc Natl Acad Sci U S A 89(13):5847-51.

## Revendications

1. Méthode de détection, non invasive prénatale, *in vitro* de l'état sain normal, de l'état de porteur sain ou de l'état de porteur malade de la mucoviscidose, à partir d'un échantillon de cellule(s) foetale(s) isolée(s) d'un échantillon maternel prélevé comprenant l'ADN à tester d'un individu, ladite méthode comprenant les étapes suivantes :
a. l'enrichissement en cellules foetales d'un échantillon maternel prélevé, pur ou dilué, susceptible de comprendre des cellules d'origine foetale ;
b. l'analyse des cellules retenues pendant l'étape a) et la sélection des cellules présumées d'origine foetale ;
c. la démonstration par analyse génétique, de l'origine foetale d'une ou plusieurs cellules sélectionnées à l'étape b et,
d. sur l'ADN foetal de cellule(s) sélectionnée(s) à l'étape c., la recherche d'allèles du gène CFTR porteurs de la mutation ΔF508 ou d'autres mutations connues ou, la recherche d'allèles d'un locus portant un polymorphisme génotypique génétiquement lié à une mutation morbide non identifiée du gène CFTR, au moyen des étapes suivantes:
● l'amplification de l'ADN foetal au moyen de couples d'amorces choisis pour leur capacité à amplifier le locus susceptible de porter la mutation connue recherchée sur le gène CFTR ou un locus comprenant un polymorphisme génotypique génétiquement lié (linkage) à la mutation du gène CFTR lors de la ségrégation,
● l'identification sur les allèles correspondant aux fragments d'ADN amplifiés, de la présence ou de l'absence de la mutation connue recherchée du gène CFTR ou du locus polymorphique génétiquement lié au gène CFTR et
● la comparaison des allèles foetaux avec les allèles correspondant d'échantillons contrôle et la détermination à partir de l'observation des allèles amplifiés, de la détection de l'état sain normal, de l'état de porteur sain ou de l'état de porteur malade de la mucoviscidose chez l'individu testé.

2. Méthode de détection, non invasive, in vitro de l'état sain normal, de l'état de porteur sain ou de l'état de porteur malade de la mucoviscidose, à partir d'un échantillon de cellule(s) foetale(s) isolée(s) d'un échantillon maternel prélevé comprenant l'ADN à tester d'un individu, ladite méthode comprenant les étapes suivantes :
a. l'enrichissement en cellules foetales d'un échantillon maternel prélevé, pur ou dilué, susceptible de comprendre des cellules d'origine foetale;
b. l'analyse des cellules retenues pendant l'étape a) et la sélection des cellules présumées d'origine foetale séparées des autres cellules circulantes ;
c. sur l'ADN foetal de cellule(s) sélectionnée(s) à l'étape b, la recherche d'allèles du gène CFTR porteurs de la mutation ΔF508 ou d'autres mutations connues ou, la recherche d'allèles d'un locus portant un polymorphisme génotypique génétiquement lié à une mutation morbide non ΔF508 ou non identifiée du gène CFTR, au moyen des étapes suivantes :
- l'amplification de l'ADN foetal au moyen de couples d'amorces choisis pour leur capacité à amplifier le locus susceptible de porter la mutation connue recherchée sur le gène CFTR ou un locus comprenant un polymorphisme génotypique génétiquement lié au gène CFTR lors de sa ségrégation,
- l'identification sur les allèles correspondant aux fragments d'ADN amplifiés, de la présence ou de l'absence de la mutation connue recherchée du gène CFTR ou du locus polymorphique génétiquement lié au gène CFTR.

3. Méthode de détection, non invasive prénatale, *in vitro* de l'état sain normal, de l'état de porteur sain ou de l'état de porteur malade de la mucoviscidose selon la revendication 1, dans laquelle les amorces utilisées aux étapes c et d sont capables d'amplifier une petite quantité d'ADN, ou dans laquelle les amorces utilisées aux étapes c et d sont capables d'amplifier l'ADN d'une cellule unique.

4. Méthode de détection, non invasive prénatale, *in vitro* de l'état sain normal, de l'état de porteur sain ou de l'état de porteur malade de la mucoviscidose selon la revendication 3, dans laquelle les amorces informatives utilisées à l'étape c. sont dérivées de la séquence du chromosome 7.

5. Méthode de détection, non invasive prénatale, de l'état sain normal, de l'état de porteur sain ou de l'état de porteur malade de la mucoviscidose selon la revendication 1 ou 3, dans laquelle, l'anomalie du gène CFTR recherchée n'est pas connue et les étapes c et d sont réalisées dans les conditions suivantes :
● l'amplification selon les étapes c. et d. est confondue et réalisée au moyen d'amorces informatives d'amplification capables d'amplifier un locus comprenant un polymorphisme génotypique génétiquement lié (linkage) au gène CFTR lors de la ségrégation, et
● la comparaison avec les échantillons contrôles à l'étape d. comporte la comparaison des allèles identifiés de l'ADN foetal avec les allèles correspondants paternels, les allèles correspondants maternels et les allèles correspondants d'un enfant de la fratrie affecté par la mucoviscidose.

6. Méthode de détection, non invasive prénatale, de l'état sain normal, de l'état de porteur sain ou de l'état de porteur malade de la mucoviscidose selon l'une quelconque des revendications 1 à 5, dans laquelle l'étape c. comprend ou consiste en l'identification, sur une préparation d'ADN dérivée du génome d'une cellule foetale unique collectée, d'un ou plusieurs marqueurs de polymorphisme génétique ou d'une combinaison de ces marqueurs, démontrant la contribution bi-parentale à l'ADN de ladite cellule et en conséquence, l'origine foetale de ladite au moins une cellule.

7. Méthode de détection, non invasive prénatale, de l'état sain normal, de l'état de porteur sain ou de porteur malade de la mucoviscidose selon l'une quelconque des revendications 1 à 6, dans laquelle des cellules retenues pendant l'étape a) sont collectées individuellement notamment par microdissection, ou dans laquelle des cellules retenues pendant l'étape a) sont collectés, puis analysées in situ au cours de l'étape b), sans collecte individuelle des cellules.

8. Méthode de détection, non invasive prénatale, de l'état sain normal, de l'état de porteur sain ou de l'état de porteur malade de la mucoviscidose selon l'une quelconque des revendications 1 à 7, dans laquelle l'étape a) consiste en la filtration d'un échantillon maternel prélevé, pur ou dilué, susceptible de comprendre des cellules d'origine foetale, de façon à concentrer sur un filtre, selon leur taille, certaines cellules y compris des cellules d'origine foetale et, l'étape b) consiste en l'analyse des cellules retenues sur le filtre et la sélection des cellules présumées d'origine foetale.

9. Méthode de détection, non invasive prénatale, de l'état sain normal, de l'état de porteur sain ou de l'état de porteur malade de la mucoviscidose selon l'une quelconque des revendications précédentes, **caractérisée en ce que** ladite étape d. est réalisée sur le pool de l'ADN de plusieurs cellules sélectionnées individuellement à ladite étape c.

10. Polynucléotide utilisable comme amorce pour amplifier une séquence d'ADN d'un échantillon biologique **caractérisé en ce qu'**il est choisi parmi des polynucléotides dont la séquence comprend ou consiste en l'une des séquences suivantes :
- F : 5'-AAAAACCCTGGCTTATGC-3' SEQ ID NO : 1 ; R : 5'-AGCTACCATAGGGCTGGAGG-3' SEQ ID NO : 2 ;
- F : 5'-GGAATCTGTTCTGGCAATGGAT-3' SEQ ID NO : 5 ; R : 5'-TTGCAATGAGCCGAGATCCTG-3' SEQ ID NO : 6 ;
- F: 5'-CAGATGCTCGTTGTGCACAA-3' SEQ ID NO: 9 ; R: 5'-ATACCATTTACGTTTGTGTGTG-3' SEQ ID NO : 10;
- F: 5'-TGACAGTGCAGCTCATGGTC-3' SEQ ID NO : 13 ; R: 5'-GGTCATTGGTCAAGGGCTGCT-3' SEQ ID NO : 14;
- F: 5'-TTGACATTCTTCTGTAAGGAAGA-3' SEQ ID NO : 17 ; R : 5'-AGGCTTGCCAAAGATATTAAAAG-3' SEQ ID NO : 18 ;
- F : 5'-TTGTGAATAGTGCTGCAATG-3' SEQ ID NO : 21 ; R: 5'-ATGTACACTGACTTGTTTGAG-3' SEQ ID NO : 22 ;
- F: 5'- AAGTAATTCTCCTGCCTCAG-3' (SEQ ID NO: 29) ; R: 5'-AGCTACTTGCAGTGTAACAGCATTT -3' (SEQ ID NO: 30) ;
- F: 5'- GAATTATAACCGTAACTGATTC-3' (SEQ ID NO: 33) ; R:5'-GAGATAATGCTTGTCTGACTTC-3' (SEQ ID NO: 34) ;
- F : 5'-TGGAGCCTTCAGAGGGTAAA-3' SEQ ID NO : 25 ; R : 5'-TGCATAATCAAAAAGTTTTCACA-3' SEQ ID NO : 26 ;
- F : 5'-TCTGTTCTCAGTTTTCCTGG-3' SEQ ID NO : 27 ; R : 5'-TCTTACCTCTTCTAGTTGGC-3' SEQ ID NO : 28, ou un oligonucléotide dont la séquence présente une identité d'au moins 60% avec l'une de ces séquences.

11. Polynucléotide présentant au moins 60 % d'identité, de préférence 80 %, de manière préférée 95 % d'identité ou plus, avec une séquence selon la revendication 10.

12. Utilisation d'amorces dans le cadre d'une méthode de détection, non invasive prénatale, *in vitro* de l'état sain normal, de l'état de porteur sain ou de l'état de porteur malade de la mucoviscidose à partir de l'ADN génomique de cellules foetales isolées de l'échantillon maternel prélevé, **caractérisée en ce que** les amorces sont choisies parmi :
- F : 5'-AAAAACCCTGGCTTATGC-3' SEQ ID NO: 1 ; R: 5'-AGCTACCATAGGGCTGGAGG-3' SEQ ID NO : 2 ;
- F : 5'-GGAATCTGTTCTGGCAATGGAT-3' SEQ ID NO : 5 ; R : 5'-TTGCAATGAGCCGAGATCCTG-3' SEQ ID NO : 6;
- F: 5'-CAGATGCTCGTTGTGCACAA-3' SEQ ID NO :9 ; R: 5'-ATACCATTTACGTTTGTGTGTG-3' SEQ ID NO :10 ;
- F: 5'-TGACAGTGCAGCTCATGGTC-3' SEQ ID NO :13, R: 5'-GGTCATTGGTCAAGGGCTGCT-3' SEQ ID NO :14 ;
- F: 5'-TTGACATTCTTCTGTAAGGAAGA-3' SEQ ID NO :17 ; R: 5'-AGGCTTGCCAAAGATATTAAAAG-3' SEQ ID NO :18 ;
- F : 5'-TTGTGAATAGTGCTGCAATG-3' SEQ ID NO :21 ; R : 5'-ATGTACACTGACTTGTTTGAG-3' SEQ ID NO :22 ;
- F: 5'- AAGTAATTCTCCTGCCTCAG-3' (SEQ ID NO: 29) ; R: 5'-AGCTACTTGCAGTGTAACAGCATTT -3' (SEQ ID NO: 30) ;
- F: 5'- GAATTATAACCGTAACTGATTC-3' (SEQ ID NO: 33) ; R:5'-GAGATAATGCTTGTCTGACTTC-3' (SEQ ID NO: 34) ;
- F: 5'-CTTGGGGACTGAACCATCTT-3' SEQ ID NO :3 ; R: 5'-AGCTACCATAGGGCTGGAGG-3' SEQ ID NO :4 ;
- F: 5'-AAAGGCCAATGGTATATCCC-3' SEQ ID NO :7 ; R: 5'-GCCCAGGTGATTGATAGTGC-3' SEQ ID NO :8 ;
- F: 5'-GATCCCAAGCTCTTCCTCTT-3' SEQ ID NO :11 ; R : 5'-ACGTTTGTGTGTGCATCTGT-3' SEQ ID NO :12 ;
- F: 5'-GGATAAACATAGAGCGACAGTTC-3' SEQ ID NO :15 ; R : 5'-AGACAGAGTCCCAGGCATT-3' SEQ ID NO :16 ;
- F: 5'-CCCTCTCAATTGTTTGTCTACC-3' SEQ ID NO :19 ; R : 5'-GCAAGAGATTTCAGTGCCAT-3' SEQ ID NO :20 ;
- F : 5'-ATGTACATGTGTCTGGGAAGG-3' SEQ ID NO :23 ; R : 5'-TTCTCTACATATTTACTGCCAACA-3' SEQ ID NO :24 ;
- F: 5'- CCTTGGGCCAATAAGGTAAG-3' (SEQ ID NO: 31 ) ; R : 5'-AGCTACTTGCAGTGTAACAGCATTT-3' (SEQ ID NO: 32) ;
- F: 5'- CTGATTCATAGCAGCACTTG -3' (SEQ ID NO: 35) ; R:5'-AAAACATTTCCATTACCACTG-3' (SEQ ID NO: 36) ;
- F : 5'-TGGAGCCTTCAGAGGGTAAA-3' (SEQ ID NO :25) ; R : 5'-TGCATAATCAAAAAGTTTTCACA-3' (SEQ ID NO :26) ;
- F : 5'-TCTGTTCTCAGTTTTCCTGG-3' (SEQ ID NO :27) ; R : 5'-TCTTACCTCTTCTAGTTGGC-3' (SEQ ID NO :28).

13. Kit pour le diagnostic pré-natal non invasif la détection, non invasive prénatale de l'état sain normal, de l'état de porteur sain ou de l'état de porteur malade de la mucoviscidose comprenant une ou plusieurs amorces choisies parmi :
- F : 5'-GGAATCTGTTCTGGCAATGGAT-3' SEQ ID NO :5 ; R : 5'-TTGCAATGAGCCGAGATCCTG-3' SEQ ID NO :6 ;
- F : 5'-CAGATGCTCGTTGTGCACAA-3' SEQ ID NO :9 ; R : 5'-ATACCATTTACGTTTGTGTGTG-3' SEQ ID NO :10 ;
- F: 5'-TGACAGTGCAGCTCATGGTC-3' SEQ ID NO :13, R: 5'-GGTCATTGGTCAAGGGCTGCT-3' SEQ ID NO :14 ;
- R : 5'-AGGCTTGCCAAAGATATTAAAAG-3' SEQ ID NO :18 ;
- F : 5'-TTGTGAATAGTGCTGCAATG-3' SEQ ID NO :21 ; R : 5'-ATGTACACTGACTTGTTTGAG-3' SEQ ID NO :22 ;
- F: 5'- AAGTAATTCTCCTGCCTCAG-3' (SEQ ID NO: 29) ; R : 5'-AGCTACTTGCAGTGTAACAGCATTT -3' (SEQ ID NO: 30) ;
- F: 5'- GAATTATAACCGTAACTGATTC-3' (SEQ ID NO: 33) ; R:5'-GAGATAATGCTTGTCTGACTTC-3' (SEQ ID NO: 34) ;
- F: 5'- CCTTGGGCCAATAAGGTAAG-3' (SEQ ID NO: 31) ; R : 5'-AGCTACTTGCAGTGTAACAGCATTT-3' (SEQ ID NO: 32) ;
- F: 5'- CTGATTCATAGCAGCACTTG -3' (SEQ ID NO: 35) ; R:5'-AAAACATTTCCATTACCACTG-3' (SEQ ID NO: 36),
ou un oligonucléotide dont la séquence présente une identité d'au moins 60% avec l'une de ces séquences, et le cas échéant des réactifs pour effectuer l'amplification de l'ADN et/ou des instructions pour effectuer la détection de l'état de porteur sain ou de porteur malade de la mucoviscidose.

14. Composition comprenant des cellules provenant d'un prélèvement maternel contenant des cellules foetales, placées sur un support et capables d'être utilisées pour réaliser la méthode de détection de l'état sain normal, de l'état de porteur sain ou de l'état de porteur malade de la mucoviscidose, selon les revendications 1 à 3.

15. Filtre poreux comprenant des cellules eucaryotes isolées selon la revendication 14 et permettant la détection ou l'absence de la mutation connue recherchée du gène CFTR ou du locus polymorphique génétiquement lié au gène CFTR.
